# EUROPEAN PATENT SPECIFICATION

(11) **EP 3 816 628 B1**
(45) Date of publication and mention of the grant of the patent: **16.08.2023**
(21) Application number: 19827564.6
(22) Date of filing: 21.06.2019
(51) Int. Cl.: G01N 33/574, G01N 33/66

(54) **PANCREATIC CANCER DETERMINATION MARKER**
MARKER ZUR BESTIMMUNG VON PANKREASKREBS
MARQUEUR DE DÉTERMINATION DU CANCER DU PANCRÉAS

(30) Priority: 26.06.2018 JP 2018120945
(43) Date of publication of application: 05.05.2021
(73) Proprietor: FUJIFILM Corporation, Tokyo 106-8620 (JP); Public University Corporation Yokohama City University, Yokohama-shi, Kanagawa 236-0027 (JP)
(72) Inventor: KAWASAKI, Nana, Yokohama-shi, Kanagawa 230-0045 (JP); OHTA, Yuki, Kawasaki-shi, Kanagawa-ken 213-0002 (JP); ICHIKAWA, Yasushi, Yokohama-shi, Kanagawa 236-0004 (JP); TERAUCHI, Yasuo, Yokohama-shi, Kanagawa 236-0004 (JP); SHIBATA, Wataru, Yokohama-shi, Kanagawa-ken 236-0027 (JP)
(74) Representative: Winter, Brandl - Partnerschaft mbB
(86) International application number: PCT/JP2019/024634
(87) International publication number: WO 2020/004244

(56) References cited:
- WO-A1-2009/136506
- WO-A1-2011/034182
- WO-A1-2013/172105
- JP-A- 2003 004 748
- JP-A- 2008 541 060
- SONG NIE ET AL: "Glycoprotein Biomarker Panel for Pancreatic Cancer Discovered by Quantitative Proteomics Analysis", JOURNAL OF PROTEOME RESEARCH, vol. 13, no. 4, 10 March 2014 (2014-03-10) , pages 1873-1884, XP055463005, ISSN: 1535-3893, DOI: 10.1021/pr400967x
- DAISUKE NAKATA: "Increased N-glycosylation of Asn88 in serum pancreatic ribonuclease 1 is a novel diagnostic marker for pancreatic cancer", SCIENTIFIC REPORTS, vol. 4, 22 October 2014 (2014-10-22), page 6715, XP055232102, DOI: 10.1038/srep06715
- MIYAKO NAKANO ET AL: "Site-specific analysis of N-glycans on haptoglobin in sera of patients with pancreatic cancer: A novel approach for the development of tumor markers", INTERNATIONAL JOURNAL OF CANCER, JOHN WILEY & SONS, INC, US, vol. 122, no. 10, 23 January 2008 (2008-01-23), pages 2301-2309, XP071283687, ISSN: 0020-7136, DOI: 10.1002/IJC.23364
- LIU XIAOHUI ET AL: "A new panel of pancreatic cancer biomarkers discovered using a mass spectrometry-based pipeline", BRITISH JOURNAL OF CANCER, vol. 117, no. 12, 9 November 2017 (2017-11-09), pages 1846-1854, XP055882455, London ISSN: 0007-0920, DOI: 10.1038/bjc.2017.365 Retrieved from the Internet: URL:http://www.nature.com/articles/bjc2017 365>

## Description

### Technical Field

The present invention relates to a pancreatic cancer determination marker.

### Background Art

"Sugar chain" refers to a generic name for a molecule in which monosaccharides such as glucose, galactose, mannose, fucose, xylose, N-acetylglucosamine, N-acetylgalactosamine, and sialic acid and derivatives thereof are bound in a chain form by glycosidic bonds.

Sugar chains are mainly present on the cell surface in the form of glycoconjugates bound to proteins and lipids in vivo, and are involved in physiological functions, such as cell proliferation, bacterial or viral infection, nerve elongation, inflammation, and immunity.

Meanwhile, it is also known that the sugar chain changes its structure in association with diseases such as pancreatic cancer, and studies have been conducted to use the sugar chain as a marker for diagnosing a disease such as pancreatic cancer in recent years.

For example, CA19-9 (carbohydrate antigen 19-9: pancreatic cancer marker) and haptoglobin (pancreatic cancer marker) are known as a marker for diagnosing a disease such as pancreatic cancer having a sugar chain (Patent Literature 1).

Non Patent Literature 1 is directed to a glycoprotein biomarker panel for pancreatic cancer discovered by quantitative proteomics analysis.

Non Patent Literature 2 relates to increased N-glycosylation of Asn88 in serum pancreatic ribonuclease 1 as a novel diagnostic marker for pancreatic cancer.

Non Patent Literature 3 mentions site-specific analysis of N-glycans of haptoglobin in sera of patients with pancreatic cancer and Non Patent Literature 4 relates to a panel of pancreatic cancer biomarkers discovered using a mass spectrometry-based pipeline

However, these known pancreatic cancer markers having a sugar chain have not been sufficient in terms of accuracy (correctness and precision).

### Citation List

### Patent Literature

Patent Literature 1: JP2009-168470A

### Non Patent Literature

Non Patent Literature 1: Song Nie et al., "Glycoprotein Biomarker Panel for Pancreatic Cancer Discovered by Quantitative Proteomics Analysis", Journal of Proteome Research, vol. 13, no. 4, 10 March 2014, pages 1873-1884
Non Patent Literature 2: Daisuke Nakata, "Increased N-glycosylation of Asn88 in serum pancreatic ribonuclease 1 is a novel diagnostic marker for pancreatic cancer", Scientific reports, vol. 4, 22 October 2014, page 6715
Non Patent Literature 3: Myako Nakano et al., "Site-specific analysis of N-glycans of haptoglobin in sera of patients with pancreatic cancer: A novel approach for the development of tumor markers", International Journal of Cancer, vol. 122, no. 10, 23 January 2008, pages 2301-2309
Non Patent Literature 4: Liu Xiaohui et al., "A new panel of pancreatic cancer biomarkers discovered using a mass spectrometry-based pipeline", British Journal of Cancer, vol. 117, no. 12, 9 November 2017, pages 1846-1854

### Summary of Invention

### Technical Problem

The present invention has an object of providing a pancreatic cancer determination marker with a high accuracy (correctness and precision).

### Solution to Problem

The present invention has been made for the purpose of achieving the object, and provides a pancreatic cancer determination marker, a pancreatic cancer determination method and a use of a pancreatic cancer determination kit as defined in the claims:

### Advantageous Effects of Invention

According to the pancreatic cancer determination marker, the pancreatic cancer determination method using the same, and the method for acquiring data for determining a pancreatic cancer of the present invention, it is possible to determine pancreatic cancer (diagnosis, testing) with a high accuracy (correctness and precision).

### (Brief Description of Drawings)

Fig. 1 is a graph showing average values of the peak areas of glycopeptides A, B, and C in healthy subjects (11 test samples) and pancreatic cancer patients (10 test samples).
Fig. 2 is a graph showing average values of the peak areas of glycopeptides D and E in healthy subjects (11 test samples) and pancreatic cancer patients (10 test samples).
Fig. 3 is a graph showing average values of the peak areas of glycopeptides F and G in healthy subjects (11 test samples) and pancreatic cancer patients (10 test samples).
Fig. 4 is a graph showing average values of the peak areas of glycopeptide H in healthy subjects (11 test samples) and pancreatic cancer patients (10 test samples).
Fig. 5 is a graph showing average values of the peak areas of glycopeptide I in healthy subjects (11 test samples) and pancreatic cancer patients (10 test samples).
Fig. 6 is a graph showing average values of the peak areas of glycopeptide J in healthy subjects (11 test samples) and pancreatic cancer patients (10 test samples).
Fig. 7 is a graph showing average values of the peak areas of glycopeptides K and L in healthy subjects (11 test samples) and pancreatic cancer patients (10 test samples).

### Description of embodiments

### <Pancreatic cancer determination marker of the present invention>

A pancreatic cancer determination marker of the present invention (hereinafter, sometimes referred to, shortly, as "marker of the present invention") contains a sugar chain represented by the formula 5 present in inter-alpha trypsin inhibitor heavy chain H3 (hereinafter, sometimes referred to, shortly, as "marker (1) of the present invention");

The marker (1) of the present invention will be described below.

### [Marker of the present invention (1)]

The marker (1) of the present invention comprises a sugar chain represented by the formula 5 present in inter-alpha trypsin inhibitor heavy chain H3, 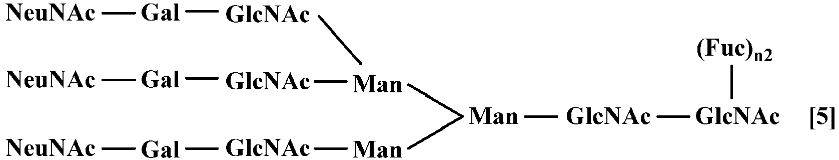 in the formula 5, n2 represents 0 or 1.

It should be noted that in the formula 5, NeuNAc represents N-acetylneuraminic acid, Gal represents galactose, GlcNAc represents N-acetylglucosamine, Man represents mannose, and Fuc represents fucose.

Hereinafter, the same meanings are used in the present specification.

It should be noted that any of the sugar chains represented by the formula 5 may be used alone or in combination of a plurality of kinds thereof as the marker (1) of the present invention.

The inter-alpha trypsin inhibitor heavy chain H3 according to the marker (1) of the present invention is one of the protease inhibitor families present in plasma, and although its function has not been clarified, it has been reported to be involved in implantation of a fetus.

In addition, the amino acid sequence of the inter-alpha trypsin inhibitor heavy chain H3 is registered in a database such as Uniprot (Accession Number: Q06033).

The inter-alpha trypsin inhibitor heavy chain H3 according to the marker (1) of the present invention includes not only those registered in the database but also mutants that can be generated in vivo and the like. For example, the inter-alpha trypsin inhibitor heavy chain H3 has 1 to 5, preferably 1 or 2 amino acids deleted, substituted or/and added, resulting from polymorphism, mutation or the like.

However, even in the case of a mutant or the like, the amino acid residue (asparagine residue) at position 580 from the N-terminus of the amino acid sequence of the inter-alpha trypsin inhibitor heavy chain H3, which is a binding site between the sugar chain represented by the formula 5 in the marker (1) of the present invention to be described later and the inter-alpha trypsin inhibitor heavy chain H3, is preferably conserved.

The sugar chain represented by the formula 5 (terminal N-acetylglucosamine) in the marker (1) of the present invention is preferably bound (N-glycosidic bond) to the amino acid residue (asparagine residue) at position 580 from the N-terminus of the amino acid sequence of inter-alpha trypsin inhibitor heavy chain H3.

The sugar chain represented by the formula 5 in the marker (1) of the present invention will be described below.

Sugar chain represented by the formula 5 present in inter-alpha trypsin inhibitor heavy chain H3 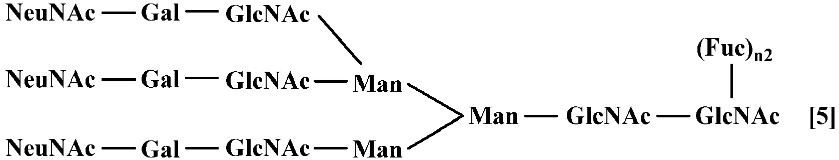
in the formula 5, n2 represents 0 or 1.
n2 in the formula 5 represents 0 or 1, and the formula 5 represents the formula 5-1 in a case where n2 is 0, and represents the formula 5-2 in a case where n2 is 1.

The sugar chain represented by the formula 5 is preferably a sugar chain represented by the formula 5-1 or a combination of a sugar chain represented by the formula 5-1 and a sugar chain represented by the formula 5-2.

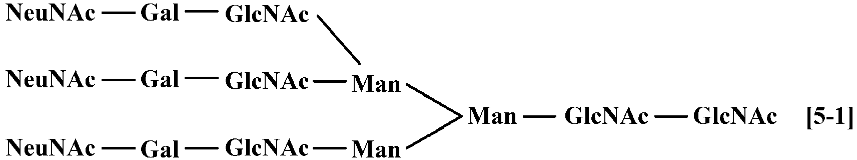

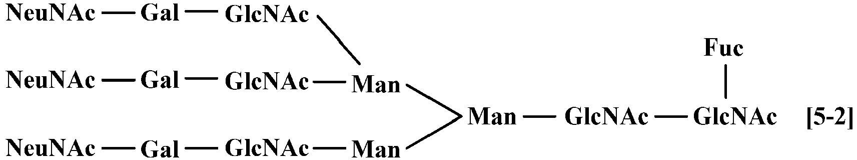

In addition, specific examples of the sugar chains represented by the formulae 5-1 and 5-2 include those represented by the formulae 5-1', 5-1", 5-2', and 5-2", and preferred is a combination of one represented by the formula 5-1' and one represented by the formula 5-1", or a combination of one represented by the formula 5-1', one represented by the formula 5-1", one represented by the formula 5-2', and one represented by the formula 5-2".

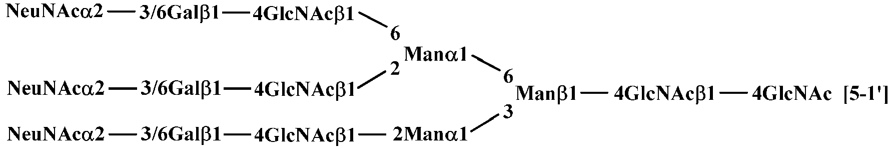

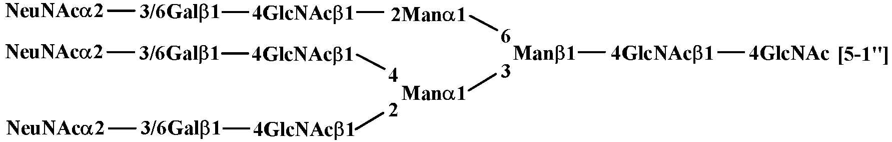

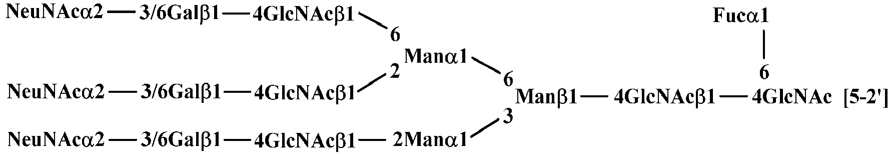

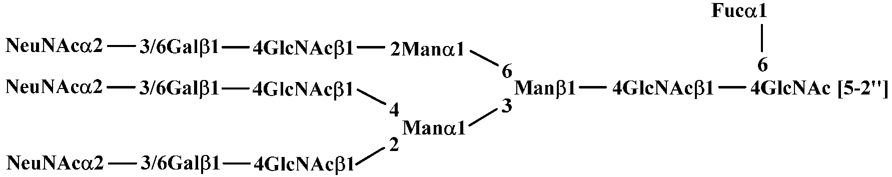

Examples of the marker (1) of the present invention, that is, the marker containing the sugar chain represented by the formula 5 present in inter-alpha trypsin inhibitor heavy chain H3 include (i) the sugar chain represented by the formula 5 itself present in (derived from) the inter-alpha trypsin inhibitor heavy chain H3, (ii) an inter-alpha trypsin inhibitor heavy chain H3 to which the sugar chain represented by the formula 5 is bound, (iii) a peptide fragment which is a part of the inter-alpha trypsin inhibitor heavy chain H3 and to which the sugar chain represented by the formula 5 is bound, and the like, of which (ii) or (iii) is preferred.

It should be noted that the peptide fragment is preferably any fragment having at least the amino acid residue (asparagine residue) at position 580 from the N-terminus of the amino acid sequence of inter-alpha trypsin inhibitor heavy chain H3, which is a binding site between the sugar chain represented by the formula 5 and inter-alpha trypsin inhibitor heavy chain H3. Specific examples of the peptide fragment include those generated in vivo and those generated as a result of treating the inter-alpha trypsin inhibitor heavy chain H3 to protease treatment with trypsin, lysyl endopeptidase, AspN, or the like. More specific examples thereof include those consisting of 2 to 50 amino acid residues, and a peptide fragment represented by SEQ ID NO: 3 (Accession number Q06033: 572 to 584) is preferred.

### [Marker (2), not covered by the subject-matter of the claims]

A marker (2) not covered by the subject-matter of the claims contains a sugar chain represented by the formula 6 present in leucine-rich alpha-2 glycoprotein.

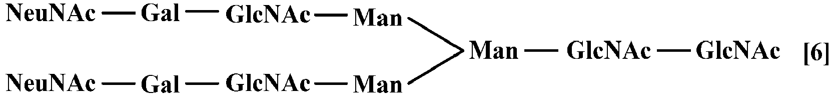

It should be noted that any of the sugar chains represented by the formula 6 may be used alone or in combination of a plurality of kinds thereof as the marker (2) (not covered by the subject-matter of the claims).

The leucine-rich alpha-2 glycoprotein (Leucine-rich alpha-2-glycoprotein) according to the marker (2), which is not covered by the subject-matter of the claims, is a protein contained in blood, and has been reported to be involved in various diseases such as inflammatory bowel disease. In addition, the amino acid sequence of leucine-rich alpha-2 glycoprotein is registered in a database such as Uniprot (Accession Number: P02750).

The leucine-rich alpha-2 glycoprotein according to the marker (2) (not covered by the subject-matter of the claims) includes not only those registered in the database but also mutants that can be generated in vivo and the like. For example, the leucine-rich alpha-2 glycoprotein has 1 to 5, preferably 1 or 2 amino acids deleted, substituted or/and added, resulting from polymorphism, mutation or the like.

However, even in the case of a mutant or the like, the amino acid residue (asparagine residue) at position 186 from the N-terminus of the amino acid sequence of the leucine-rich alpha-2 glycoprotein, which is a binding site between the sugar chain represented by the formula 6 in the marker (2) (not covered by the subject-matter of the claims) to be described later and the leucine-rich alpha-2 glycoprotein, is preferably conserved.

The sugar chain represented by the formula 6 (not covered by the subject-matter of the claims, terminal N-acetylglucosamine) in the marker (2), which is not covered by the subject-matter of the claims, is preferably bound (N-glycosidic bond) to the amino acid residue (asparagine residue) at position 186 from the N-terminus of the amino acid sequence of leucine-rich alpha-2 glycoprotein.

The sugar chain represented by the formula 6 in the marker (2), which is not covered by the subj ect-matter of the claims, will be described below.

### Sugar chain represented by the formula 6 present in leucine-rich alpha-2 glycoprotein (not covered by the subject-matter of the claims)

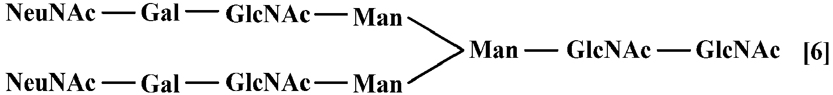

In addition, a specific and preferred example of the sugar chain represented by the formula 6 is one represented by the formula 6-1 (not covered by the subject-matter of the claims).

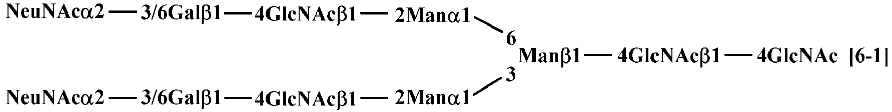

Examples of the marker (2), which are not covered by the subject-matter of the claims, that is, the marker containing the sugar chain represented by the formula 6 present in leucine-rich alpha-2 glycoprotein include (i) the sugar chain represented by the formula 6 itself present in (derived from) leucine-rich alpha-2 glycoprotein, (ii) leucine-rich alpha-2 glycoprotein to which the sugar chain represented by the formula 6 is bound, and (iii) a peptide fragment which is a part of leucine-rich alpha-2 glycoprotein and to which the sugar chain represented by the formula 6 is bound, and the like, of which (ii) or (iii) is preferred.

It should be noted that the peptide fragment is any fragment having at least the amino acid residue (asparagine residue) at position 186 from the N-terminus of the amino acid sequence of leucine-rich alpha-2 glycoprotein, which is a binding site between the sugar chain represented by the formula 6 and the leucine-rich alpha-2 glycoprotein (not covered by the subj ect-matter of the claims). Specific examples of the peptide fragment include those generated in vivo and those generated as a result of treating the leucine-rich alpha-2 glycoprotein to protease treatment with trypsin, lysyl endopeptidase, AspN, or the like. More specific examples thereof include those consisting of 2 to 50 amino acid residues, and a peptide fragment represented by SEQ ID NO: 4 (Accession number P02750: 179 to 191) is preferred.

### [Marker (3), not covered by the subject-matter of the claims]

A marker (3) not covered by the subject-matter of the claims comprises at least one sugar chain represented by the formulae 1 to 3 present in vitronectin (not covered by the subject-matter of the claims). 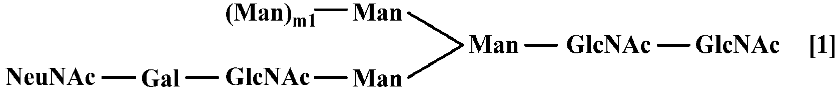
in the formula 1, m1 represents an integer of 0 to 2, 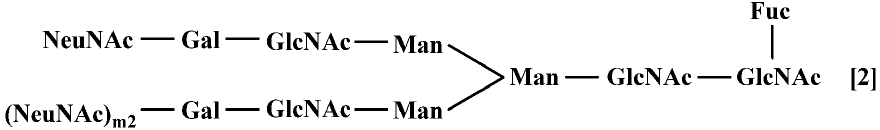
in the formula 2, m2 represents 0 or 1, 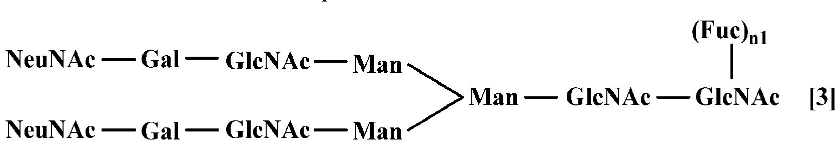
in the formula 3, n1 represents 0 or 1.

It should be noted that any of the sugar chains represented by the formulae 1 to 3 (not covered by the subject-matter of the claims) may be used alone or in combination of a plurality of kinds thereof as the marker (3). In the case of combining, a combination of the formulae 1 to 3, a combination of only the formula 1, a combination of only the formula 2, or a combination of only the formula 3 may be used.

The vitronectin according to the marker (3) (not covered by the subject-matter of the claims) is a glycoprotein present in blood, extracellular matrix and the like, and plays an important role in a living body such as tissue formation and differentiation of neurons. In addition, the amino acid sequence of the vitronectin is registered in a database such as Uniprot (Accession Number: P04004).

The vitronectin according to the marker (3) (not covered by the subject-matter of the claims) includes not only those registered in the database but also mutants that can be generated in vivo and the like. For example, the vitronectin has 1 to 5, preferably 1 or 2 amino acids deleted, substituted or/and added, resulting from polymorphism, mutation or the like.

However, even in the case of a mutant or the like, the amino acid residue (asparagine residue) at position 169 from the N-terminus of the amino acid sequence of the vitronectin, which is a binding site between the sugar chains represented by the formulae 1 to 3 in the marker (3), not covered by the subject-matter of the claims, to be described later and the vitronectin, is preferably conserved.

The sugar chains represented by the formulae 1 to 3 (terminal N-acetylglucosamine) in the marker (3) are preferably bound (N-glycosidic bond) to the amino acid residue (asparagine residue) at position 169 from the N-terminus of the amino acid sequence of vitronectin, of which more preferred is one represented by the formula 1 not covered by the subject-matter of the claims).

The sugar chains represented by the formulae 1 to 3 in the marker (3) will be described below (not covered by the subject-matter of the claims).

### Sugar chain represented by the formula 1 (not covered by the subject-matter of the claims) present in vitronectin

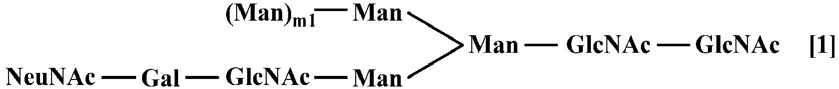

in the formula 1, m1 represents an integer of 0 to 2.
m1 in the formula 1 represents an integer of 0 to 2, and the formula 1 represents the formula 1-1 in a case where m1 is 0, represents the formula 1-2 in a case where m1 is 1, and represents the formula 1-3 in a case where m1 is 2 (not covered by the subject-matter of the claims).

The formula I, which is not covered by the subject-matter of the claims, is preferably represented by the formula 1-1, that is, that in a case where m1 is 0.

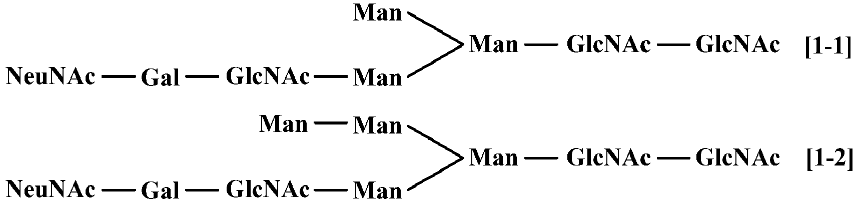

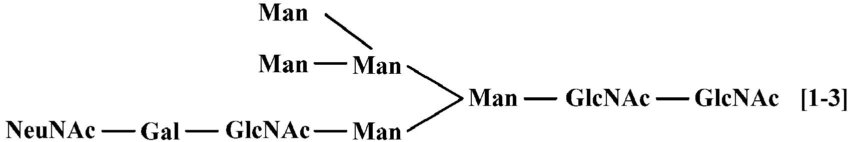

In addition, specific examples of the sugar chains represented by the formulae 1-1, 1-2, and 1-3 include those represented by the formulae 1-1', 1-2' and 1-3', of which preferred is one represented by the formula 1-1' (not covered by the subject-matter of the claims). 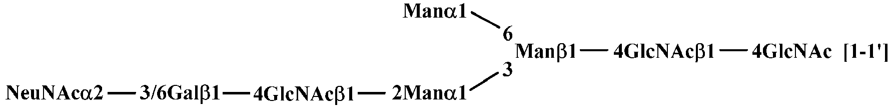 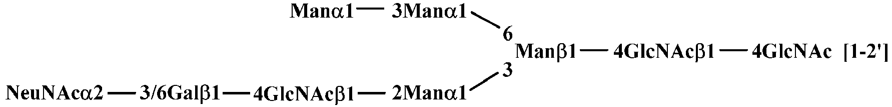 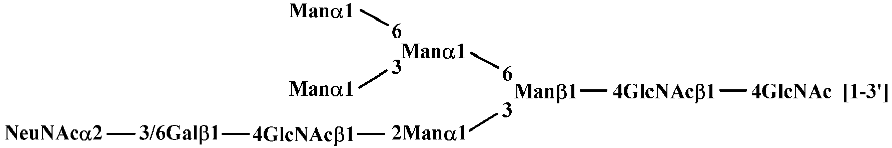 in the formulae 1-1', 1-2', and 1-3', α2-3/6 represents an α2-3 glycosidic bond or an α2-6 glycosidic bond, and hereinafter, the same meaning is used herein.

Sugar chain represented by the formula 2 present in vitronectin 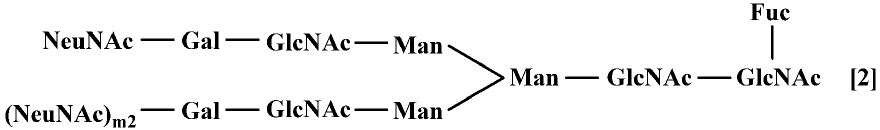
in the formula 2, m2 represents 0 or 1 (not covered by the subject-matter of the claims).
m2 in the formula 2 represents 0 or 1, and the formula 2 represents the formula 2-1 in a case where m2 is 0, and represents the formula 2-2 in a case where m2 is 1.

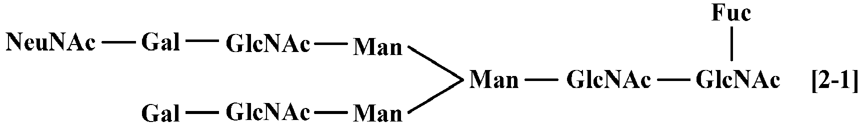

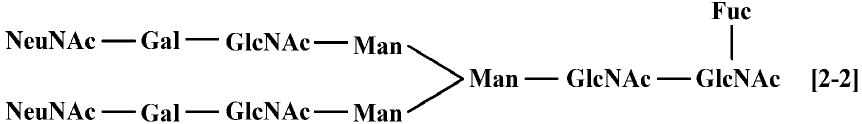

In addition, specific examples of the sugar chains represented by the formulae 2-1 and 2-2 include those represented by the formulae 2-1', 2-1", and 2-2', and one represented by the formula 2-1', and a combination of one represented by the formula 2-1' and one represented by the formula 2-1" are preferred (not covered by the subject-matter of the claims).

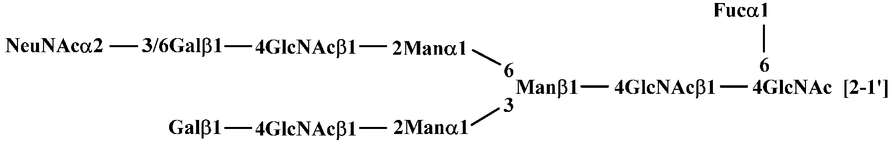

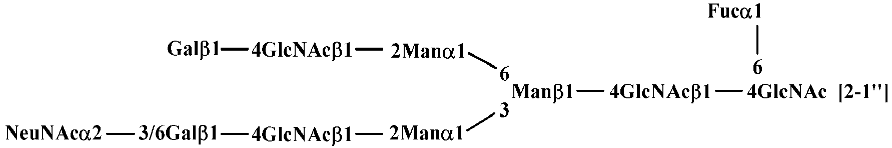

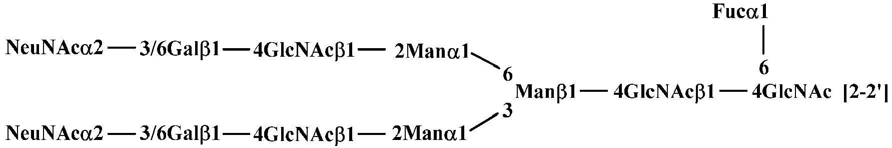

Sugar chain represented by the formula 3 present in vitronectin 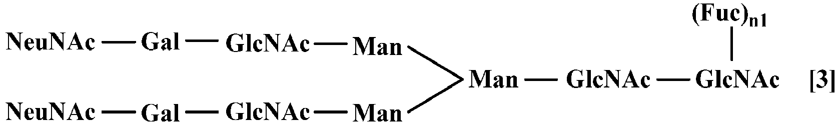
in the formula 3, n1 represents 0 or 1 (not covered by the subject-matter of the claims).
n1 in the formula 3 represents 0 or 1, and the formula 3 represents the formula 3-1 in a case where n1 is 0, and represents the formula 3-2 in a case where n1 is 1.

The formula 3 is preferably represented by the formula 3-2, that is, that in a case where n1 is 1 (not covered by the subject-matter of the claims).

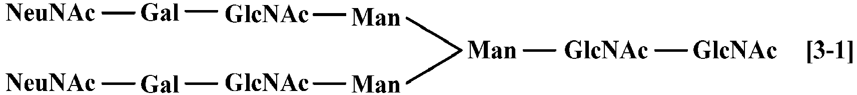

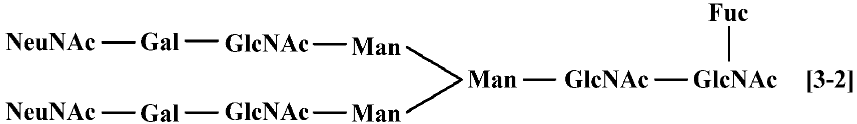

In addition, specific examples of the sugar chains represented by the formulae 3-2, and 3-1 include those represented by the formulae 3-1' and 3-2', of which preferred is one represented by the formula 3-2' (not covered by the subject-matter of the claims).

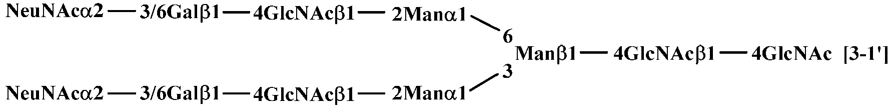

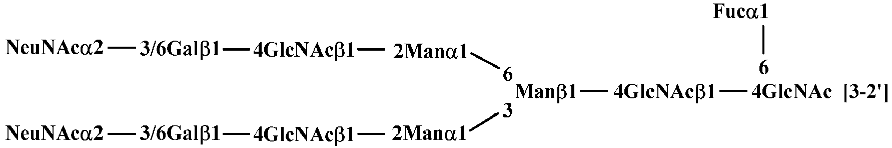

Examples of the marker (3) (not covered by the subject-matter of the claims), that is, the marker containing at least one sugar chain represented by the formulae 1 to 3 present in vitronectin include (i) the at least one sugar chain represented by the formulae 1 to 3 itself present in (derived from) vitronectin, (ii) vitronectin to which the at least one sugar chain represented by the formulae 1 to 3 is bound, (iii) a peptide fragment which is a part of vitronectin and to which the at least one sugar chain represented by the formulae 1 to 3 is bound, and the like, of which (ii) or (iii) is preferred.

It should be noted that the peptide fragment is preferably any fragment having at least the amino acid residue (asparagine residue) at position 169 from the N-terminus of the amino acid sequence of vitronectin, which is a binding site between the sugar chains represented by the formulae 1 to 3 and vitronectin. Specific examples of the peptide fragments include those generated in vivo and those generated as a result of treating the vitronectin to protease treatment with trypsin, lysyl endopeptidase, AspN, or the like. More specific examples thereof include those consisting of 2 to 50 amino acid residues, and a peptide fragment represented by SEQ ID NO: 1 (Accession number P04004: 169 to 176) is preferred.

### [Marker (4), not covered by the subject-matter of the claims]

A marker (4) (not covered by the subject-matter of the claims) comprises a sugar chain represented by the formula 4 present in complement C4-A.

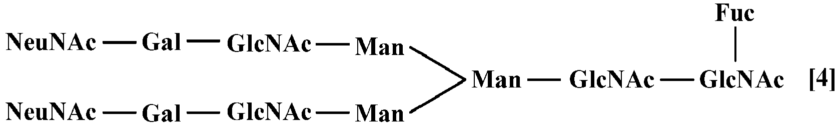

It should be noted that any of the sugar chains represented by the formula 4 may be used alone or in combination of a plurality of kinds thereof as the marker (4) (not covered by the subject-matter of the claims).

The complement C4-A according to the marker (4) is one of plasma proteins (not covered by the subject-matter of the claims), is produced in hepatocytes and the like, and plays an important role in defense against infection with bacteria and the like. In addition, the amino acid sequence of the complement C4-A is registered in a database such as Uniprot (Accession Number: P0C0L4).

The complement C4-A according to the marker (4) (not covered by the subject-matter of the claims) includes not only those registered in the database but also mutants that can be generated in vivo and the like. For example, the complement C4-A has 1 to 5, preferably 1 or 2 amino acids deleted, substituted or/and added, resulting from polymorphism, mutation or the like.

However, even in the case of a mutant or the like, the amino acid residue (asparagine residue) at position 1328 from the N-terminus of the amino acid sequence of the complement C4-A, which is a binding site between the sugar chain represented by the formula 4 in the marker (4) (not covered by the subject-matter of the claims) to be described later and the complement C4-A, is preferably conserved.

The sugar chain represented by the formula 4 (terminal N-acetylglucosamine) in the marker (4) is preferably bound (N-glycosidic bond) to the amino acid residue (asparagine residue) at position 1328 from the N-terminus of the amino acid sequence of complement C4-A (not covered by the subject-matter of the claims).

The sugar chain represented by the formula 4 in the marker (4) (not covered by the subject-matter of the claims) will be described below.

Sugar chain represented by the formula 4 (not covered by the subject-matter of the claims) present in complement C4-A

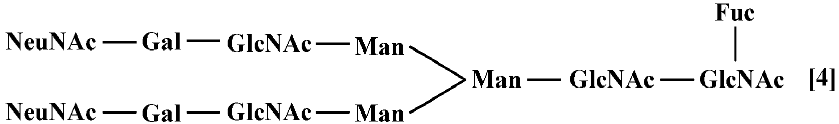

In addition, a specific and preferred example of the sugar chain represented by the formula 4 is represented by the formula 4-1 (not covered by the subject-matter of the claims).

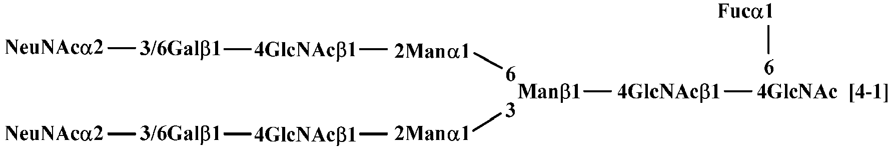

Examples of the marker (4) (not covered by the subject-matter of the claims), that is, the marker containing the sugar chain represented by the formula 4 present in complement C4-A, include (i) the sugar chain represented by the formula 4 itself present in (derived from) complement C4-A, (ii) complement C4-A to which the sugar chain represented by the formula 4 is bound, and (iii) a peptide fragment which is a part of the complement C4-A and to which the sugar chain represented by the formula 4 is bound, and the like, of which (ii) or (iii) is preferred.

It should be noted that the peptide fragment is preferably any fragment having at least the amino acid residue (asparagine residue) at position 1328 from the N-terminus of the amino acid sequence of complement C4-A, which is a binding site between the sugar chain represented by the formula 4 and complement C4-A. Specific examples of the peptide fragments include those generated in vivo and those generated as a result of treating the complement C4-A to protease treatment with trypsin, lysyl endopeptidase, AspN, or the like. More specific examples thereof include those consisting of 2 to 50 amino acid residues, and a peptide fragment represented by SEQ ID NO: 2 (Accession number P0C0L4: 1326 to 1336) is preferred.

### [Marker (5), not covered by the subject-matter of the claims]

A marker (5), which is not covered by the subject-matter of the claims, comprises a sugar chain represented by the formula 3 present in thyroxine-binding globulin. 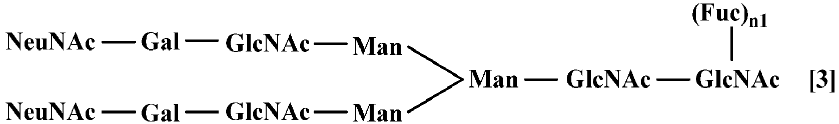 in the formula 3, n1 represents 0 or 1.

It should be noted that any of the sugar chains represented by the formula 3 may be used alone or in combination of a plurality of kinds thereof as the marker (5) (not covered by the subject-matter of the claims).

The thyroxine-binding globulin according to the marker (5) (not covered by the subject-matter of the claims) is a protein that binds to thyroxine, and has been reported to be involved in the transport of thyroid hormone and the like. In addition, the amino acid sequence of the thyroxine-binding globulin is registered in a database such as Uniprot (Accession Number: P05543).

The thyroxine-binding globulin according to the marker (5) (not covered by the subject-matter of the claims) includes not only those registered in the database but also mutants that can be generated in vivo and the like. For example, the thyroxine-binding globulin has 1 to 5, preferably 1 or 2 amino acids deleted, substituted or/and added, resulting from polymorphism, mutation or the like.

However, even in the case of a mutant or the like, the amino acid residue (asparagine residue) at position 36 from the N-terminus of the amino acid sequence of the thyroxine-binding globulin, which is a binding site between the sugar chain represented by the formula 3 in the marker (5) to be described later and the thyroxine-binding globulin, is preferably conserved (not covered by the subject-matter of the claims).

The sugar chain represented by the formula 3 (terminal N-acetylglucosamine) in the marker (5), which is not covered by the subject-matter of the claims, is preferably bound (N-glycosidic bond) to the amino acid residue (asparagine residue) at position 36 from the N-terminus of the amino acid sequence of thyroxine-binding globulin.

It should be noted that the sugar chain represented by the formula 3 in the marker (5), which is not covered by the subject-matter of the claims, is as described above, and its specific examples are the same, but preferred examples include those represented by the formula 3-1, and more preferred examples include those represented by the formula 3-1' (not covered by the subject-matter of the claims).

Examples of the marker (5), which is not covered by the subject-matter of the claims, that is, the marker containing the sugar chain represented by the formula 3 present in thyroxine-binding globulin, include (i) the sugar chain represented by the formula 3 itself present in (derived from) thyroxine-binding globulin, (ii) thyroxine-binding globulin to which the sugar chain represented by the formula 3 is bound, (iii) a peptide fragment which is a part of the thyroxine-binding globulin and to which the sugar chain represented by the formula 3 is bound, and the like, of which (ii) or (iii) is preferred.

It should be noted that the peptide fragment is preferably any fragment having at least the amino acid residue (asparagine residue) at position 36 from the N-terminus of the amino acid sequence of thyroxine-binding globulin, which is a binding site between the sugar chain represented by the formula 3 and thyroxine-binding globulin (not covered by the subject-matter of the claims). Specific examples of the peptide fragments include those generated in vivo and those generated as a result of treating the thyroxine-binding globulin to protease treatment with trypsin, lysyl endopeptidase, AspN, or the like. More specific examples thereof include those consisting of 2 to 50 amino acid residues, and a peptide fragment represented by SEQ ID NO: 5 (Accession number P05543: 27 to 41) is preferred (not covered by the subject-matter of the claims).

The marker of the present invention is (i) the sugar chain represented by the formula 5 present in the inter-alpha trypsin inhibitor heavy chain H3, more preferably (ii) the sugar chain represented by the formula 5 present in inter-alpha trypsin inhibitor heavy chain H3, and particularly preferably (iii) the sugar chain represented by the formula 5 bound to the amino acid residue (asparagine residue) at position 580 from the N-terminus of the amino acid sequence of inter-alpha trypsin inhibitor heavy chain H3.

### [Pancreatic cancer according to the present invention]

The pancreatic cancer according to the present invention is a cancer arising from the pancreas.

Specific examples of the pancreatic cancer according to the present invention include exocrine pancreatic cancers such as pancreatic invasive ductal carcinoma, pancreatic acinar cell carcinoma, and intraductal papillary mucinous tumor, and endocrine pancreatic cancers such as neuroendocrine tumor.

### <Pancreatic cancer determination method of the present invention>

A pancreatic cancer determination method of the present invention (hereinafter, sometimes referred to, shortly, as determination method of the present invention) comprises measuring an amount of a sugar chain selected from the following (1) in a sample (hereinafter, sometimes referred to, shortly, as measurement step according to the present invention), and determining pancreatic cancer based on the obtained measurement result (hereinafter, sometimes referred to, shortly, as determination step according to the present invention).
(1) a sugar chain represented by the formula 5 present in inter-alpha trypsin inhibitor heavy chain H3 (marker (1) of the present invention).

It should be noted that the determination method of the present invention may be performed using the marker (1) of the present invention.

### [Sample according to the present invention]

A sample according to the present invention may be derived from a test animal, and example thereof include biological samples such as serum, plasma, whole blood, urine, saliva, cerebrospinal fluid, tissue fluid, sweat, tears, amniotic fluid, bone marrow fluid, pleural effusion, ascites, indirect fluid, aqueous humor, and vitreous humor, of which preferred are blood-derived samples such as serum, plasma, and whole blood, and more preferred is serum.

Examples of the test animal include mammals such as human, monkey, mouse, rat, dog, cat, pig, rabbit, chimpanzee, of which preferred is human, monkey, mouse, or rat, and more preferred is human.

A method of obtaining (collecting) a sample according to the present invention from a test animal is not particularly limited, and may be performed by, for example, obtaining (collecting) the sample from the test animal based on a method known per se, and if necessary, separation, concentration, purification, or the like may be performed according to a method known per se.

It should be noted that the sample may be one immediately after being obtained (collected) from the test animal or may be a sample that has been stored. The sample may be stored by any method commonly used in this field.

### [Measurement step according to the present invention]

The measurement step according to the present invention comprises measuring an amount of a sugar chain selected from the following (1):
(1) a sugar chain represented by the formula 5 present in inter-alpha trypsin inhibitor heavy chain H3.

It should be noted that the measurement step according to the present invention may be performed by measuring the marker (1) of the present invention.

The measurement of the amount of the sugar chain selected from the following (1) in the measurement step according to the present invention is to measure one selected from the marker (1) of the present invention, and specific examples thereof include (i) measurement of the amount of sugar chain itself present in (derived from) the protein in the marker of the present invention, (ii) measurement of the amount of protein to which the sugar chain is bound in the marker of the present invention, and (iii) measurement of the amount of a peptide fragment which is a part of the protein to which the sugar chain is bound in the marker of the present invention, of which (ii) or (iii) is preferred.

The protein in the marker (1) of the present invention means inter-alpha trypsin inhibitor heavy chain H3.

It should be noted that the "amount" may be an absolute value such as volume or mass, or a relative value such as a concentration, an ionic intensity, an absorbance, a fluorescence intensity, a turbidity, a value calculated from peak areas, or the like.

The measurement method in the measurement step according to the present invention may be any method commonly used in this field, and specific examples thereof include (A) a method of using a substance having an affinity for the marker of the present invention (for example, antibody, lectin, etc.), and (B) a method of utilizing mass spectrometry, of which preferred is (A).

(A) and (B) will be specifically described below.

### (A) Method of using substance having affinity for marker of the present invention

Specific examples of the method of using a substance having an affinity for the marker of the present invention include a method according to an immunological assay such as enzyme-linked immunosorbent assay (ELISA), enzyme immunoassay (EIA), radioimmunoassay (RIA), fluorescence immunoassay (FIA), chemiluminescent enzyme immunoassay (CLEIA), electrochemiluminescence immunoassay (ECLEIA), immunoturbidimetry, immunonephelometry, latex agglutination test, immunochromatography, Western blotting, luminescent oxygen channeling immunoassay (LOCI), and liquid-phase binding assay-electrokinetic analyte transport assay (LBA-EATA).

Specifically, the method according to an immunological assay may be performed, for example, by bringing a substance having an affinity for the marker of the present invention into contact with the marker of the present invention, forming a complex of the substance having an affinity for the marker of the present invention and the marker of the present invention, and measuring the amount of the complex.

Examples of the substance having an affinity for the marker of the present invention include an antibody that specifically binds to the protein (or peptide fragment) in the marker of the present invention, and a lectin or antibody that specifically binds to the sugar chain in the marker of the present invention.

Specific examples of the antibody that specifically binds to the protein (or peptide fragment) in the marker of the present invention include an anti-inter-alpha trypsin inhibitor heavy chain H3 antibody in the case of the marker (1) of the present invention, an anti-leucine-rich alpha-2 glycoprotein antibody.

The antibody may be a polyclonal antibody or a monoclonal antibody, and these may be used alone or in combination.

The antibody may be an antibody fragment such as Fab, F(ab'2), Fv, and sFv, or a synthetic antibody such as diabody, triabody, and tetrabody.

In addition, the antibody used may be a commercially available antibody or one prepared according to a method known per se.

It should be noted that in a case where the antibody is prepared according to a method known per se, it may be performed according to a method disclosed in, for example, "Immunoassay" (edited by Biochemical Assay Society of JAPAN, Kodansha, 2014).

In addition, the antibody may be labeled with a labeling substance. Specific examples of the labeling substance include enzymes such as horseradish peroxidase (HRP), bovine small intestinal alkaline phosphatase, and β-galactosidase, radioactive isotopes such as ^{99m}Tc, ¹³¹I, ¹²⁵I, ¹⁴C, ³H, ³²P, and ³⁵S, fluorescent substances such as fluorescein, fluorescein isothiocyanate (FITC), 4-methylumbelliferone, rhodamine or derivatives thereof, luminescent substances such as luciferin, luminol, and ruthenium complexes, substances having absorption in the ultraviolet region such as phenol, naphthol, anthracene or derivatives thereof, substances having a property as a spin-labeling agent represented by a compound having an oxyl group such as 4-amino-2,2,6,-tetramethylpiperidine-1-oxyl, dyes such as HiLyte series dyes, Alexa series dyes, and CyDye series dyes, gold colloids, and nanoparticles such as quantum dots.

It should be noted that a method of binding the labeling substance to the antibody may be performed according to a method known per se.

Specific examples of the lectin that specifically binds to the sugar chain in the marker of the present invention include: Phaseolus vulgaris Leucoagglutinin (PHA-L) or any other lectin that binds to GlcNAc (N-acetylglucosamine) of the sugar chain represented by the formula 5 in the case of the marker (1) of the present invention.

The lectin may be labeled with a labeling substance, and the labeling substance is as described above for the antibody that specifically binds to a protein (or peptide fragments) in the marker of the present invention, and specific examples thereof are also the same. In addition, a method of binding the labeling substance to the lectin may be performed according to a method known per se.

Specific examples of the antibody that specifically binds to the sugar chain in the marker of the present invention include: an antibody that specifically binds to the sugar chain represented by the formula 5 in the case of the marker (1) of the present invention.

The antibody may be labeled with a labeling substance, and the labeling substance is as described above for the antibody that specifically binds to a protein (or peptide fragments) in the marker of the present invention, and specific examples thereof are also the same.

In addition, a method of binding the labeling substance to the lectin may be performed according to a method known per se.

The method of measuring the amount of the complex of the substance having an affinity for the marker of the present invention of the present invention and the marker may be any method as long as it can measure the amount of the complex, and specific examples thereof include a method of detecting a signal derived from the labeling substance bound to the substance having an affinity for the marker of the present invention, a method of utilizing a property derived from the complex of the substance having an affinity for the marker of the present invention and the marker of the present invention, of which preferred is the method of detecting a signal derived from the labeling substance bound to the substance having an affinity for the marker of the present invention.

Specifically, the method of detecting a signal derived from the labeling substance bound to the substance having an affinity for the marker of the present invention may be performed, for example, by a signal derived from the labeling substance bound to the substance having an affinity for the marker of the present invention a method known per se. For example, in a case where the labeling substance is an enzyme, the measurement may be performed according to a conventional method of immunoassay, for example, a method disclosed in "Enzyme Immunoassay" (Protein, Nucleic Acid and Enzyme, Separate Volume No. 31, edited by Tsunehiro Kitagawa, Toshio Nanbara, Akio Tsuji, and Eiji Ishikawa, pp. 51-63, Kyoritsu Shuppan Co., Ltd., 1987), and in a case where the labeling substance is a radioactive substance, the measurement may be performed, for example, according to a conventional method which is performed by RIA, and by appropriately selecting and using a measurement apparatus such as an immersion GM counter, a liquid scintillation counter, a well-type scintillation counter, or a counter for HPLC, depending on the type and intensity of radiation generated by the radioactive substance (see, for example, "Course on Experimental Medical Chemistry", Vol. 8, supervised by Yuichi Yamamura, the 1st edition, Nakayama Shoten, 1971). In addition, in a case where the labeling substance is a fluorescent substance, the measurement may be performed according to a conventional method which is performed by FIA using a measurement apparatus such as a fluorophotometer, for example according to a method disclosed in "Illustrative Description of Fluorescent Antibody", Akira Kawaoi, the 1st edition, Soft Science Inc., 1983; and in a case where the labeling substance is a luminescent substance, the measurement may be performed according to a conventional method using a measurement apparatus such as a photo counter, for example, according to a method disclosed in "Enzyme Immunoassay" (Protein, Nucleic Acid and Enzyme, Separate Volume No. 31, edited by Tsunehiro Kitagawa, Toshio Nanbara, Akio Tsuji, and Eiji Ishikawa, pp. 252-263, Kyoritsu Shuppan Co., Ltd., 1987). Further, in a case where the labeling substance is a substance which has absorption in the ultraviolet region, the measurement may be performed by a conventional method using a measurement apparatus such as a spectrophotometer; and in a case where the labeling substance has a property of spin, the measurement may be performed by a conventional method using electron spin resonance equipment, for example, according to a method disclosed in "Enzyme Immunoassay" (Protein, Nucleic Acid and Enzyme, Separate Volume No. 31, Tsunehiro Kitagawa, Toshio Nanbara, Akio Tsuji, and Eiji Ishikawa, pp. 264-271, Kyoritsu Shuppan Co., Ltd., 1987).

Specific examples of the method of utilizing a property derived from the complex of the substance having an affinity for the marker of the present invention and the marker of the present invention include a method such as a homogeneous immunoassay system such as a surface plasmon resonance method.

It should be noted that a specific scheme of the method may be performed according to a method known per se.
(A) The method of using a substance having an affinity for the marker of the present invention will be described more specifically below.

It should be noted that in the following description, the protein (or peptide fragment) in the marker of the present invention is referred to as "target protein (or target peptide fragment)", and the sugar chain in the marker of the present invention is referred to as "target sugar chain".

For example, in the case of measuring the marker (1) of the present invention, inter-alpha trypsin inhibitor heavy chain H3 is the "target protein (or target peptide fragment)", and the sugar chain represented by the formula 5 is the "target sugar chain.

Specific examples of the method (A) of using a substance having an affinity for the marker of the present invention include a method including the following steps 1 and 2:
(Step 1) a step of separating a target protein (marker of the present invention) to which a target sugar chain is bound from a sample; and
(Step 2) a step of measuring an amount of the target protein to which the target sugar chain is bound separated in the step 1.

The step 1 may be any method as long as it can separate the target protein to which the target sugar chain is bound from the sample, and specific examples thereof include a method of separating the target protein present in the sample to which the target sugar chain is bound and other components from each other. Specifically, such a method may be performed by, for example, forming a complex (substance that specifically binds to the target protein - the marker of the present invention - substance that specifically binds to the target sugar chain) of the target protein to which the target sugar chain is bound and substances having an affinity using two or more kinds of substances having an affinity, that is, a substance that specifically binds to the target sugar chain and a substance that specifically binds to the target protein, and separating the target protein to which the target sugar chain is bound by a method known per se.

It should be noted that in a case where the substances having an affinity labeled with a labeling substance are used, the separation of the target protein to which the target sugar chain is bound may be rephrased as the separation of the complex of the substances having an affinity labeled with a labeling substance and the marker of the present invention, and in this case, a complex of the substances having an affinity labeled with a labeling substance and components other than the marker of the present invention or/and the substances having an affinity labeled with a labeling substance in a free state may be separated. Separation from components that do not contain the substances having an affinity labeled with a labeling substance is unnecessary.

In addition, the substance having an affinity that specifically binds to the sugar chain and the substance having an affinity that specifically binds to the protein are as described above, and specific examples thereof are also the same.

The step 2 may be any method as long as it can measure the target protein to which the target sugar chain is bound separated in the step 1, and specific examples are as described above.

The method may be performed by subjecting the total protein (containing target protein) in the sample to peptide fragmentation in advance before performing the step 1, separating the target peptide fragment to which the target sugar chain is bound in the step 1, and measuring the target peptide fragment to which the target sugar chain is bound in the step 2.

Specifically, the fragmentation treatment may be performed by, for example, adding a protease, such as trypsin, lysyl endopeptidase, and AspN, to the sample before performing the step 1, forming a complex (substance that specifically binds to the target peptide fragment - the marker of the present invention - substance that specifically binds to the target sugar chain) of the target peptide fragment to which the target sugar chain is bound and substances having an affinity using a substance that binds to the target sugar chain and a substance that binds to the target peptide fragment and separating the complex as described above in the step 1, and then measuring an amount of the complex.

In addition, in a case where the total protein is fragmented, the fragmentation may be followed by concentration with a column such as a lectin column. The concentration may be performed according to a method known per se, and a column such as a commercially available lectin column may be used.

Further, in the method, instead of the step 2, the target sugar chain may be separated from the target protein to which the target sugar chain is bound separated in the step 1, and only the target sugar chain may be measured.

Specifically, for example, the target protein to which the target sugar chain is bound separated in the step 1 may be treated with glycanase, hydrazine, or the like to separate the target sugar chain, and then only the amount of the sugar chain may be measured as described above.

### (B) Method of utilizing mass spectrometry

Specific examples of a method of utilizing mass spectrometry include a method of using a liquid chromatography mass spectrometer (LC/MS), a capillary electrophoresis mass spectrometer (CE/MS), a gas chromatography mass spectrometer (GC/MS), a liquid chromatography tandem mass spectrometer (LC/MS/MS), or the like.

Specifically, the method may be performed by, for example, separating components in a sample in a separation section having a chromatograph such as a liquid chromatograph, ionizing various separated components in a mass spectrometry section, and further separating the components for each mass-to-charge ratio (m/z).

It should be noted that the specific techniques of the method of utilizing mass spectrometry may be performed according to a method known per se or a technique disclosed in WO2014/038524, WO2017/019588, WO2018/034346, and the like.

In the method of utilizing the mass spectrometry, the total protein may be extracted from the sample in advance, and specifically, for example, the total protein may be precipitated by using a solvent such as acetone, methanol, ethanol, trichloroacetic acid, and aqueous hydrochloric acid to the sample.

In addition, the total protein may be fragmented before or after extracting the total protein from the sample as necessary, and in a case where the fragmentation is performed, concentration by a column such as a lectin column may also be performed following the fragmentation. The fragmentation and concentration are as described above.

### [Preferred measurement step]

The measurement step according to the present invention is preferably performed by measuring (i) an amount of the sugar chain represented by the formula 5 present in the inter-alpha trypsin inhibitor heavy chain H3, more preferably (ii) an amount of the sugar chain represented by the formula 5 present in inter-alpha trypsin inhibitor heavy chain H3, and even more preferably (iii) an amount of the sugar chain represented by the formula 5 bound to the amino acid residue (asparagine residue) at position 580 from the N-terminus of the amino acid sequence of inter-alpha trypsin inhibitor heavy chain H3.

In addition, in the case of performing the measurement, preferred is a method using a substance having an affinity for the marker of the present invention; more preferred is a method according to an immunological assay such as ELISA, EIA, RIA, FIA, CLEIA, ECLEIA, immunoturbidimetry, immunonephelometry, latex agglutination test, immunochromatography, Western blotting, LOCI, and LBA-EATA; even more preferred is a method of bringing a substance having an affinity for the marker of the present invention into contact with the marker of the present invention, forming a complex of the substance having an affinity for the marker of the present invention and the marker of the present invention, and measuring the amount of the complex; and particularly preferred is a method of bringing a substance having an affinity for the marker of the present invention into contact with the marker of the present invention, forming a complex of the substance having an affinity for the marker of the present invention and the marker of the present invention, and detecting a signal derived from a labeling substance bound to the complex.

### [Determination step according to the present invention]

The determination step according to the present invention is a step of determining pancreatic cancer based on the measurement result obtained by the measurement step according to the present invention.

Specifically, the determination step according to the present invention is performed using, for example, a value obtained by the measurement step according to the present invention using a sample derived from a test animal (hereinafter, sometimes referred to, shortly, as value derived from test animal) and a preset reference value (cutoff value, etc.).

That is, (i) in a case where the value derived from the test animal is equal to or higher than the preset reference value (cutoff value, etc.), a determination such as "the test animal is likely to suffer from a pancreatic cancer or the test animal is highly likely to suffer from a pancreatic cancer" can be made, and (ii) in a case where the value derived from the test animal is lower than the preset reference value (cutoff value, etc.), a determination such as "the test animal is unlikely to suffer from a pancreatic cancer or the test animal is highly unlikely to suffer from a pancreatic cancer" can be made.

In addition, in another aspect, (i) in a case where the value derived from the test animal is equal to or lower than the preset reference value (cutoff value, etc.), a determination such as "the test animal is likely to suffer from a pancreatic cancer or the test animal is highly likely to suffer from a pancreatic cancer" can be made, and (ii) in a case where the value derived from the test animal is higher than the preset reference value (cutoff value, etc.), a determination such as "the test animal is unlikely to suffer from a pancreatic cancer or the test animal is highly unlikely to suffer from a pancreatic cancer" can be made.

It should be noted that the reference value (cutoff value, etc.) can be determined based on a statistical analysis such as receiver operating characteristic (ROC) curve analysis using a measurement value obtained by the measurement step according to the present invention using a sample from an animal suffering from a pancreatic cancer and a value obtained by the measurement step according to the present invention using a sample from a healthy animal (hereinafter, sometimes referred to, shortly, as value derived from healthy animal).

In addition, the sensitivity or/ specificity of the reference value (cutoff value, etc.) is, for example, 60% or more, preferably 70% or more, more preferably 80% or more, and even more preferably 90% or more.

In another aspect, the value derived from the test animal is compared with the value derived from the healthy animal, and (i) in a case where the value derived from the test animal is higher than the value derived from the healthy animal, a determination such as "the test animal is likely to suffer from a pancreatic cancer or the test animal is highly likely to suffer from a pancreatic cancer" can be made, and (ii) in a case where there is no significant difference between the value derived from the test animal and the value derived from the healthy animal, a determination such as "the test animal is unlikely to suffer from a pancreatic cancer or the test animal is highly unlikely to suffer from a pancreatic cancer" can be made.

In yet another aspect, the value derived from the test animal at a certain time point is compared with the value derived from the test animal at a different time point with respect to the same test animal, and, the presence or absence of the value or/and the degree of increase/decrease are evaluated, thereby enabling diagnosis of the degree of progression and malignancy of pancreatic cancer, postoperative prognosis, or the like. That is, (i) in a case where there is an increase in the value, a determination can be made that the pathological condition has progressed to pancreatic cancer (or the malignancy of pancreatic cancer has increased) or there is a sign of the pathological condition progressing to pancreatic cancer (or there is a sign of increasing malignancy of pancreatic cancer), and (ii) in a case where there is a decrease in the value, a determination can be made that improvement has been made in the pathological condition of pancreatic cancer or there is a sign of improvement in the pathological condition of pancreatic cancer.

It should be noted that in the case of measuring a plurality of kinds of the markers of the present invention in the measurement step according to the present invention, the determination step according to the present invention may be performed as described above using the obtained plurality of values, and a determination with higher accuracy (correctness and precision) can be made.

### <Method for acquiring data for determining pancreatic cancer of the present invention>

A method for acquiring data for determining a pancreatic cancer of the present invention (hereinafter, sometimes referred to, shortly, as method for acquiring data of the present invention) comprises measuring an amount of a sugar chain selected from the following (1) in a sample:
(1) a sugar chain represented by the formula 5 present in inter-alpha trypsin inhibitor heavy chain H3.

That is, the method for acquiring data of the present invention comprises measuring a marker of the present invention, in other words, the markers (1) of the present invention.

Examples of the data in the method for acquiring data of the present invention include (i) a value of the amount of the marker of the present invention obtained by the method for acquiring the data of the present invention, (ii) a value obtained by further subjecting the value to a multivariate analysis such as multiple logistic regression analysis, discriminant analysis, Poisson regression analysis, multiple regression analysis, Cox proportional hazard model, or path analysis, (iii) data indicating the magnitude relationship between the amount of the marker of the present invention obtained by the method for acquiring data of the present invention and the reference value (cutoff value, etc.) (Comparison Results), and (iv) data indicating that the test animal is likely to suffer from a pancreatic cancer or the test animal is highly likely to suffer from a pancreatic cancer, of which preferred is (i) or (iii), and more preferred is (i).

It should be noted that the marker of the present invention, the measurement of the marker, the pancreatic cancer, and the sample in the method for acquiring data of the present invention are as described in <Marker of the present invention> and <Determination method of the present invention>, and specific examples and preferred examples thereof are also the same.

### <Use of a Pancreatic cancer determination kit of the present invention>

The use of a pancreatic cancer determination kit of the present invention (hereinafter, sometimes referred to, shortly, as kit of the present invention) comprises a substance having an affinity for a sugar chain represented by the formula 5 present in inter-alpha trypsin inhibitor heavy chain H3.

Use of the kit of the present invention comprises a substance having an affinity for the marker (1) of the present invention.

It should be noted that the marker of the present invention, and the pancreatic cancer in the kit of the present invention are as described in <Marker of the present invention> and <Determination method of the present invention>, and specific examples and preferred examples thereof are also the same.

The substance having an affinity specifically binds to the sugar chain or protein (or peptide fragment) in the marker of the present invention, and examples thereof include lectins and antibodies.

The substance having an affinity is as described in <Determination method of the present invention>, and the same applies to specific examples and preferred examples.

Use of the kit of the present invention may further contain a reagent commonly used in this field, such as a reagent for peptide fragmentation such as trypsin, lysyl endopeptidase, and AspN, a concentration column such as a lectin column, a solvent for protein extraction, a buffer, a detergent, a reaction accelerator, sugars, a protein, salts, a stabilizer such as a surfactant, a preservative, a solution for diluting a sample, an immobilized solid phase such as a lectin-immobilized solid phase, an antibody-immobilized solid phase, and an antigen-immobilized solid phase, a secondary antibody labeled with a labeling substance or fragment of the secondary antibody, a reagent for detecting a labeling substance, and the like, which does not impair the stability of coexisting reagents and the like. In addition, the concentration and pH thereof may also be within the ranges commonly used in this field.

The immobilized solid phase such as the lectin-immobilized solid phase, the antibody-immobilized solid phase, and the antigen-immobilized solid phase may be any solid phase in which a substance (lectin, antibody or fragment of the antibody, etc.) having an affinity that specifically binds to the protein (or peptide fragment) or the sugar chain in the marker of the present invention is immobilized on a material such as magnetic particles such as magnetic silica particles, polystyrene, polycarbonate, polyvinyl toluene, polypropylene, polyethylene, polyvinyl chloride, nylon, polymethacrylate, gelatin, agarose, cellulose, polyethylene terephthalate, glass, ceramic, or the like.

It should be noted that the material for the immobilized solid phase such as the lectin-immobilized solid phase, the antibody-immobilized solid phase, and the antigen-immobilized solid phase may be a material produced by a method known per se or a material commercially available. For example, in a case where magnetic particles such as the magnetic silica particles are produced by a method known per se, it can be produced by the method disclosed in WO2012/173002.

The secondary antibody or antibody fragment thereof labeled with a labeling substance is an antibody or antibody fragment thereof that binds to the immobilized substance having an affinity (such as lectin, antibody or fragment of the antibody, etc.), respectively.

It should be noted that the method of binding the labeling substance and the labeling substance in the secondary antibody or antibody fragment thereof labeled with the labeling substance is as described in <Determination method of the present invention>, and preferred examples and specific examples thereof are also the same.

In a case where the substance having an affinity such as lectin, an antibody or fragment of the antibody is labeled with a labeling substance, the reagent for detecting a labeling substance detects the label in the substance having an affinity such as the lectin, the antibody or fragment of the antibody labeled with a labeling substance or/and the label in the labeled secondary antibody or fragment of the secondary antibody, and examples thereof include a substrate for measuring an absorbance such as tetramethylbenzidine or orthophenylenediamine, a fluorescent substrate such as hydroxyphenylpropionic acid or hydroxyphenylacetic acid, a luminescent substance such as luminol, a reagent for measuring an absorbance such as 4-nitrophenylphosphate, and a fluorescent substrate such as 4-methylumbelliferyl phosphate.

Use of the kit of the present invention may further include a manual for performing the determination method of the present invention or/and the method for acquiring data, or the like. The "manual" refers to an instruction manual, an attached document, a pamphlet (leaflet), or the like of the reagent according to the present invention in which the features, principles, operating procedures, determination procedures, and the like of the determination method and the method for acquiring data of the present invention are substantially described in text or/and diagrams.

As described above, according to the use of the kit of the present invention, the determination method of the present invention or/and the method for acquiring data of the present invention can be performed easily, in a short period of time and with a high precision.

### <Apparatus for determining pancreatic cancer according to the present invention>

An apparatus for determining a pancreatic cancer according to the present invention (hereinafter, sometimes referred to, shortly, as determination apparatus according to the present invention) comprises at least (1) a measurement section. It may further comprise (2) a determination section, (3) an output section, and (4) an input section.
(1) The measurement section in the determination apparatus according to the present invention is configured to measure an amount of the sugar chain selected from the markers (1) to (5) of the present invention in a sample. Specific examples thereof include various mass spectrometers used in the measurement step according to the present invention, and measurement apparatuses such as apparatuses used in a method according to an immunological assay.
   It should be noted that, if necessary, (1) the measurement section may also be configured to calculate the amounts of the markers (1) to (5) of the present invention based on the measurement values.
(2) The determination section in the determination apparatus according to the present invention is configured to determine pancreatic cancer based on the result obtained by (1) the measurement section.
(3) The output section in the determination apparatus according to the present invention is configured to output the result obtained by (1) the measurement section or/and the result obtained by (2) the determination section.
(4) The input section in the determination apparatus according to the present invention is configured to send a signal for operating (1) the measurement section to (1) the measurement section in response to an operation by an operator.

It should be noted that the measurement, determination, or the like performed by (1) the measurement section and (2) the determination section of the determination apparatus according to the present invention are as described in <Determination method of the present invention>, and preferred examples and specific examples thereof are also the same.

According to the determination apparatus of the present invention, the determination method of the present invention or/and the method for acquiring data of the present invention can be performed easily, in a short period of time and with a high precision.

### <Method of assisting the determination of pancreatic cancer according to the present invention>

A method of assisting the determination of pancreatic cancer according to the present invention (hereinafter, sometimes referred to, shortly, as assisting method according to the present invention) comprises measuring an amount of a sugar chain selected from the markers (1) to (5) of the invention in a sample and assisting the determination of pancreatic cancer based on the obtained measurement result.

The assisting method according to the present invention can be used as a method for assisting a doctor or the like in diagnosing pancreatic cancer.

It should be noted that the sample, the marker, the measurement, the determination, and the like in the assisting method according to the present invention are as described in <Determination method of the present invention>, and preferred examples and specific examples thereof are also the same.

### <Method of determining and treating pancreatic cancer according to the present invention>

A method of determining and treating pancreatic cancer according to the present invention (hereinafter, sometimes referred to, shortly, as treatment method according to the present invention) comprises measuring an amount of a sugar chain selected from the markers (1) to (5) of the present invention in a sample, determining pancreatic cancer based on the obtained measurement result, and providing appropriate treatment to a patient determined to have a risk of pancreatic cancer or a high risk of pancreatic cancer based on the results of the determination.

It should be noted that the sample, the marker, the measurement, the determination, and the like in the treatment method according to the present invention are as described in <Determination method of the present invention>, and preferred examples and specific examples thereof are also the same.

Specific examples of appropriate treatment in the treatment method according to the present invention include surgical treatments such as pancreaticoduodenectomy, distal pancreatectomy, total pancreatectomy, and bypass surgery, radiotherapy such as chemoradiotherapy, and pharmacotherapy performed by administering a drug such as UFT (trade name).

Hereinafter, the present invention will be described more specifically based on Examples, but the present invention is not limited to the Examples.

### Examples

### Example 1. Screening of Pancreatic Cancer Markers

### [(1) Test sample (sample)]

Serum derived from pancreatic cancer patients (10 test samples) and serum from healthy subjects (11 test samples) provided by Yokohama City University Center Hospital and the biobank of Yokohama City University Advanced Medical Research Center were used as samples.

### [(2) Preparation of glycopeptide]

In a High-Select Top 14 Abundant Protein Depletion Resin (manufactured by ThermoFisher Scientific Inc.), 10 µL each of the serum derived from pancreatic cancer patients and healthy subjects in (1) was placed and incubated at 25°C for 10 min to remove contaminating proteins such as albumin and immunoglobulin present in the serum. Then, 40 µL of acetone was added to each, and the mixture was incubated at -20°C for 16 hours and then centrifuged at 14000 rpm. After removing the supernatant, 50 µL of 8 M urea-containing 50 mM Tris-hydrochloric acid buffer (pH 8.0) was added to each precipitate and dissolved, and the protein concentration thereof was measured with a protein assay BCA kit (manufactured by Fujifilm Wako Pure Chemical Corporation). After the measurement, 1 µL of 500 mM dithiothreitol was added to 20 µg of each protein sample, and the mixture was incubated at 37°C for 30 min. Then, reductive alkylation was performed by adding 2.8 µL of 500 mM iodoacetamide thereto and incubating the mixture for 30 min at 37°C (it should be noted that the reductive alkylation was terminated by adding 0.5 µL of 500 mM dithiothreitol). Thereafter, the solvent was replaced with 130 µL of 50 mM Tris-hydrochloric acid buffer (pH 8.0) with the Zeba Spin Desalting column (0.5 mL; manufactured by ThermoFisher Scientific Inc.) to remove the denaturing agent and the reducing agent. Then, Trypsin/Lys-C Mix (manufactured by Promega Corporation) was added to 1 µg of each protein sample, and the mixture was incubated at 37°C for 16 hours. Thereafter, desalting was performed using an Oasis PRiME HLB solid-phase extraction column (manufactured by Waters Corporation) and dried with a centrifugal evaporator to obtain a glycopeptide.

### [(3) Concentration of glycopeptide]

According to the following procedure, 10 µg of each glycopeptide of 21 test samples (pancreatic cancer patient: 10 test samples, healthy subject: 11 test samples) prepared in (2) was concentrated.
(A) A glycopeptide concentration column disclosed in WO 2017/195887 was loaded with 100 µL of a 0.1% TFA solution and set in a tabletop centrifuge dedicated to 200 µL chips, and the solution was passed through the column to wash a polymeric polyol layer.
(B) The column was loaded with 100 µL of an 80% acetonitrile/0.1% TFA solution and set in a tabletop centrifuge dedicated to 200 µL chips, and the solution was passed through the column to wash the solid phase extraction carrier phase.
(C) The column was loaded with 100 µL of an 80% acetonitrile/0.1% TFA solution, set in a tabletop centrifuge dedicated to 200 µL chips, and centrifuged for 2 seconds to remove air from the column.
(D) The column loaded with an 80% acetonitrile/0.1% TFA solution in (C) was loaded with 10 µg of the glycopeptide sample prepared in (2), set in a tabletop centrifuge dedicated to 200 µL chips, and centrifuged for 20 seconds.
(E) The column was loaded with 100 µL of an 80% acetonitrile/0.1% TFA solution, set in a tabletop centrifuge dedicated to 200 µL chips, and centrifuged for 20 seconds to remove impurities.
(F) (E) was repeated twice again.
(G) The column was loaded with 100 µL of a 0.1% TFA, set in a tabletop centrifuge dedicated to 200 µL chips, and centrifuged for 30 seconds to concentrate the glycopeptide in the solid phase extraction carrier phase.
(H) The column was loaded with 100 µL of an 80% acetonitrile/0.1% TFA solution, and the glycopeptide was eluted into a 1.5 mL tube using a syringe with an adapter, followed by drying with a centrifugal evaporator to obtain concentrated glycopeptide.

### [(4) Preparation of deglycosylated peptide]

In 10 µL of 50 mM sodium phosphate buffer (pH 7.4), 5 µg of the concentrated glycopeptide sample prepared in (3) was dissolved, 1 µL of 1U PNGase F (manufactured by Roche Diagnostics K.K.) was added, and the mixture was incubated at 37°C for 16 hours to cleave the N-linked sugar chain. Then, after desalting with an Oasis PRiME HLB solid phase extraction column (manufactured by Waters Corporation), the product was dried with a centrifugal evaporator to obtain a deglycosylated peptide.

### [(5) Analysis by nano liquid chromatography mass spectrometry (nanoLC/MS/MS)]

The concentrated glycopeptide prepared in (3) was dissolved in 4 µL of 0.1% formic acid to a concentration of 2.5 µg/µL, to give a glycopeptide sample. In addition, the deglycosylated peptide prepared in (4) was dissolved in 4 µL of 0.1% formic acid to a concentration of 1.25 µg/µL, to give a deglycosylated peptide sample.

The glycopeptide sample and deglycosylated peptide sample were analyzed (separation/analysis) by nano liquid chromatography mass spectrometry (nanoLC/MS/MS).

Specifically, a nano-liquid chromatograph (EASY-nLC 1000; manufactured by ThermoFisher Scientific Inc.) equipped with an analytical column (Nano HPLC capillary column; manufactured by Nikkyo Technos Co., Ltd.) was connected to a hybrid quadrupole Orbitrap mass spectrometer (Q Exactive; manufactured by ThermoFisher Scientific Inc.), 4 µL of the glycopeptide sample was injected into the analytical column, and then the glycopeptide sample was separated at a flow rate of 300 nL/min.

It should be noted that a 0.1% formic acid aqueous solution was used as the A solvent, a 0.1% formic acid acetonitrile was used as the B solvent, with a linear gradient from 0% B solvent to 35% B solvent over 0 min to 70 min and a linear gradient from 35% B solvent to 100% B solvent over 70 min to 75 min. In addition, positive ion mode (Applied voltage: 1900 V) was used throughout the measurements.

The deglycosylated peptides were separated in the same manner as above, and then the sugar chain binding site in the deglycosylated peptides was identified using Proteome Discoverer 1.4 (manufactured by ThermoFisher Scientific Inc.).

Further, the structure of the glycopeptide was confirmed from the mass and retention time of the glycopeptide based on the information.

### [(6) Results]

According to (1) to (5), glycopeptide A to L derived from vitronectin, complement C4-A, inter-alpha trypsin inhibitor heavy chain H3, leucine-rich alpha-2 glycoprotein, and thyroxine-binding globulin, respectively, were identified as shown in Table 1 below.

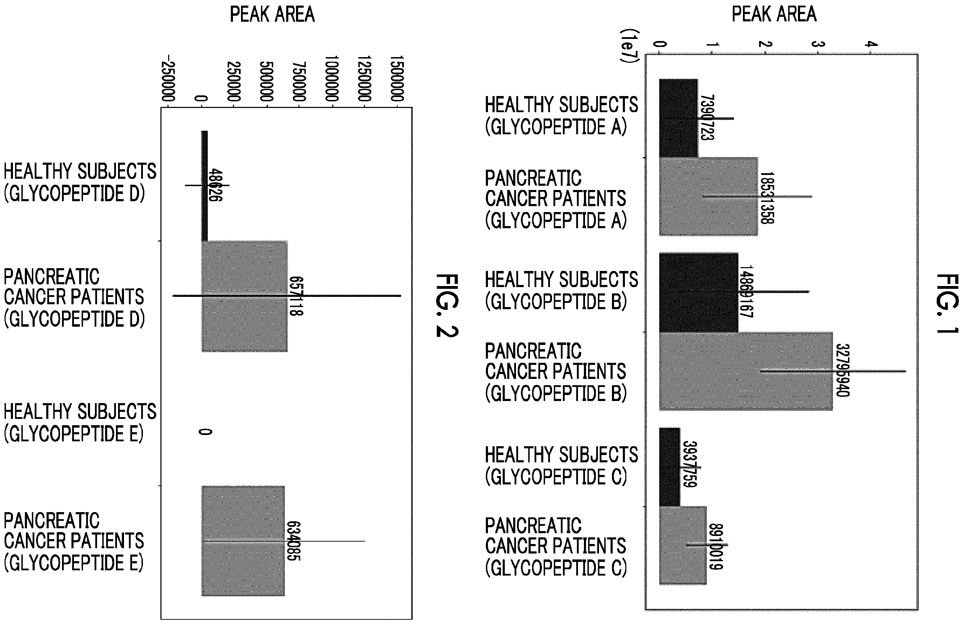

In addition, average values of the peak areas of glycopeptides A to L in healthy subjects (11 test samples) and pancreatic cancer patients (10 test samples) are shown in Figs. 1 to 7, respectively.

**[Table 1]**

| | AUC value | Glycopeptide | | | Binding site between peptide and sugar chain |
|---|---|---|---|---|---|
| | | peptide sequence | | sugar chain structure | |
| Glycopeptide A | 0,88 | vitronectin | NGSLFAFR (SEQ ID NO: 1) | Formula 1-1' | asparagine residue at position 169 from the N-terminus of the amino acid sequence of vitronectin |
| Glycopeptide B | 0,88 | | | Formula 1-2' | |
| Glycopeptide C | 0,88 | | | Formula 1-3' | |
| Glycopeptide D | 0,83 | | | Formula 2-1', Formula 2-1" | |
| Glycopeptide E | 0,83 | | | Formula 2-2' | |
| Glycopeptide F | 0,82 | | | Formula 3-1' | |
| Glycopeptide G | 0,82 | | | Formula 3-2' | |
| Glycopeptide H | 0,92 | complement C4-A | GLNVTLSSTGR (SEQ ID NO: 2) | Formula 4-1 | asparagine residue at position 1328 from the N-terminus of the amino acid sequence of complement C4-A |
| Glycopeptide I | 0,92 | inter-alpha trypsin inhibitor heavy chain H3 | NAHGEEKENL TAR (SEQ ID NO: 3) | Formula 5-1', Formula 5-1", Formula 5-2', Formula 5-2" | asparagine residue at position 580 from the N-terminus of the amino acid sequence of inter-alpha trypsin inhibitor heavy chain H3 |
| Glycopeptide J | 0,82 | leucine-rich alpha-2 glycoprotein | LPPGLLANFTLLR (SEQ ID NO: 4) | Formula 6-1 | asparagine residue at position 186 from the N-terminus of the amino acid sequence of leucine-rich alpha-2 glycoprotein |
| Glycopeptide K | 0,83 | thyroxine-binding globulin | VTACHSSQPNATL YK (SEQ ID NO: 5) | Formula 3-1' | asparagine residue at position 36 from the N-terminus of the amino acid sequence of thyroxine-binding globulin |
| Glycopeptide L | 0.80 | | | Formula 3-2' | |

It was found that the expression levels of (i) glycopeptide A having the sugar chain represented by the formula 1-1' (not covered by the subject-matter of the claims, average peak area value in healthy subjects: 7390723, average peak area value in pancreatic cancer patients: 18531358), (ii) glycopeptide B having the sugar chain represented by the formula 1-2' (not covered by the subject-matter of the claims, average peak area value in healthy subjects: 14869167, average peak area value in pancreatic cancer patients: 32795940), and (iii) glycopeptide C having the sugar chain represented by the formula 1-3' (not covered by the subject-matter of the claims, average peak area value in healthy subjects: 3937759, average peak area value in pancreatic cancer patients: 8910019), which are derived from vitronectin, are remarkably increased in pancreatic cancer patients as compared with healthy subjects, from Table 1 and Fig. 1.

In addition, it was confirmed that the sugar chains represented by the formula 1-1', which is not covered by the subject-matter of the claims, the formula 1-2' (not covered by the subject-matter of the claims), and the formula 1-3' (not covered by the subject-matter of the claims) are respectively bound to the asparagine residue at position 169 from the N-terminus of the amino acid sequence of vitronectin.

It was found that the expression levels of (i) glycopeptide D having the sugar chain represented by the formula 2-1' (not covered by the subject-matter of the claims) or the formula 2-1" (not covered by the subject-matter of the claims, average peak area value in healthy subjects: 48626, average peak area value in pancreatic cancer patients : 657118) and (ii) glycopeptide E having the sugar chain represented by the formula 2-2' (not covered by the subject-matter of the claims, average peak area value in healthy subjects: 0, average peak area value in pancreatic cancer patients: 634085), which are derived from vitronectin, are remarkably increased in pancreatic cancer patients as compared with healthy subjects, from Table 1 and Fig. 2. Particularly, it was found that (ii) glycopeptide E having the sugar chain represented by the formula 2-2' (not covered by the subject-matter of the claims) was not expressed at all in healthy subjects.

In addition, it was confirmed that the sugar chains represented by the formula 2-1' (not covered by the subject-matter of the claims), the formula 2-1" (not covered by the subject-matter of the claims), and the formula 2-2' (not covered by the subject-matter of the claims) are respectively bound to the asparagine residue at position 169 from the N-terminus of the amino acid sequence of vitronectin.

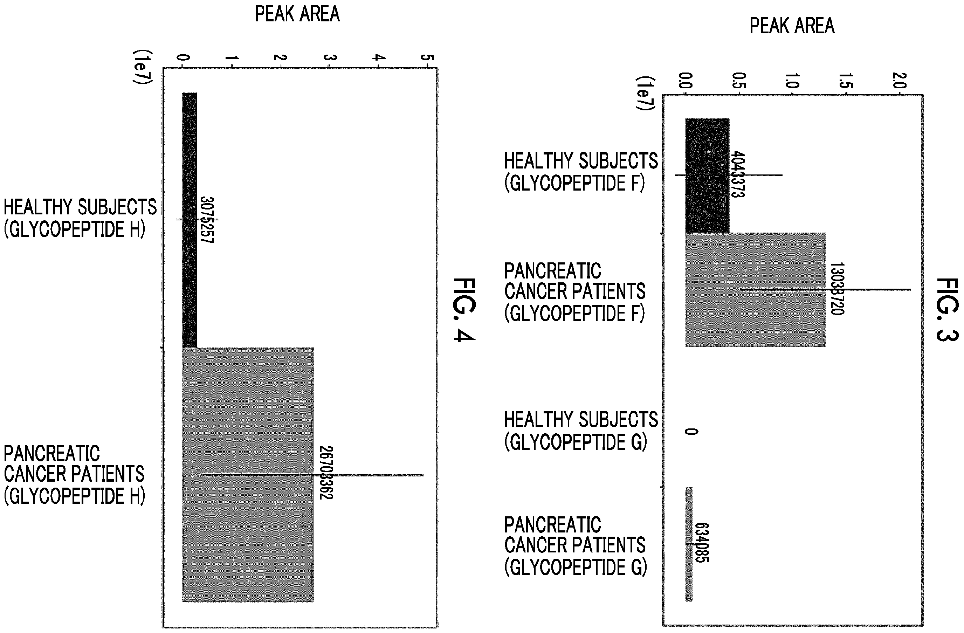

It was found that the expression levels of (i) glycopeptide F having the sugar chain represented by the formula 3-1' (not covered by the subject-matter of the claims, average peak area value in healthy subjects: 4043373, average peak area value in pancreatic cancer patients : 13038720) and (ii) glycopeptide G having the sugar chain represented by the formula 3-2' (not covered by the subject-matter of the claims, average peak area value in healthy subjects: 0, average peak area value in pancreatic cancer patients: 634085), which are derived from vitronectin, are remarkably increased in pancreatic cancer patients as compared with healthy subjects, from Table 1 and Fig. 3. Particularly, it was found that glycopeptide G having the sugar chain represented by the formula 3-2' (not covered by the subject-matter of the claims) was not expressed at all in healthy subjects.

In addition, it was confirmed that the sugar chains represented by the formula 3-1' (not covered by the subject-matter of the claims) and the formula 3-2' (not covered by the subject-matter of the claims) are respectively bound to the asparagine residue at position 169 from the N-terminus of the amino acid sequence of vitronectin.

It was found that the expression level of (i) glycopeptide H having the sugar chain represented by the formula 4-1' (not covered by the subject-matter of the claims, average peak area value in healthy subjects: 3075257, average peak area value in pancreatic cancer patients: 26703362), which is derived from complement C4-A, is remarkably increased in pancreatic cancer patients as compared with healthy subjects, from Table 1 and Fig. 4.

In addition, it was confirmed that the sugar chain represented by the formula 4-1' (not covered by the subject-matter of the claims) is bound to the asparagine residue at position 1328 from the N-terminus of the amino acid sequence of complement C4-A.

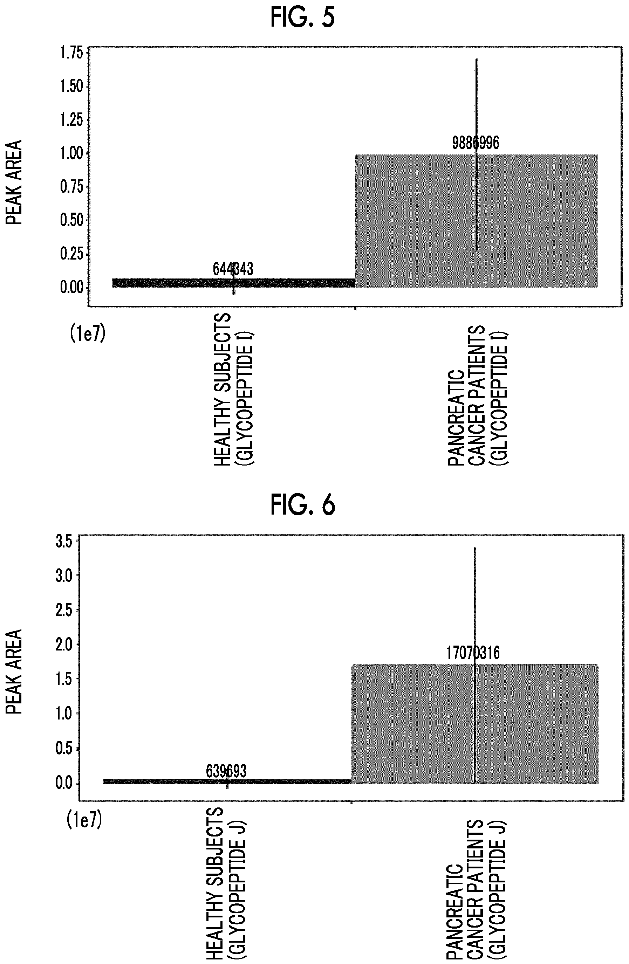

It was found that the expression level of (i) glycopeptide I having the sugar chain represented by the formula 5-1', the formula 5-1", the formula 5-2', or the formula 5-2" (average peak area value in healthy subjects: 644343, average peak area value in pancreatic cancer patients: 9886996), which is derived from inter-alpha trypsin inhibitor heavy chain H3, is remarkably increased in pancreatic cancer patients as compared with healthy subjects, from Table 1 and Fig. 5.

In addition, it was confirmed that the sugar chains represented by the formula 5-1', the formula 5-1", the formula 5-2', and the formula 5-2" are respectively bound to the asparagine residue at position 580 from the N-terminus of the amino acid sequence of inter-alpha trypsin inhibitor heavy chain H3.

It was found that the expression level of (i) glycopeptide J having the sugar chain represented by the formula 6-1' (not covered by the subject-matter of the claims, average peak area value in healthy subjects: 639693, average peak area value in pancreatic cancer patients: 17070316), which is derived from leucine-rich alpha-2 glycoprotein, is remarkably increased in pancreatic cancer patients as compared with healthy subjects, from Table 1 and Fig. 6.

In addition, it was confirmed that the sugar chain represented by the formula 6-1 (not covered by the subject-matter of the claims) was bound to the asparagine residue at position 186 from the N-terminus of the amino acid sequence of leucine-rich alpha-2 glycoprotein.

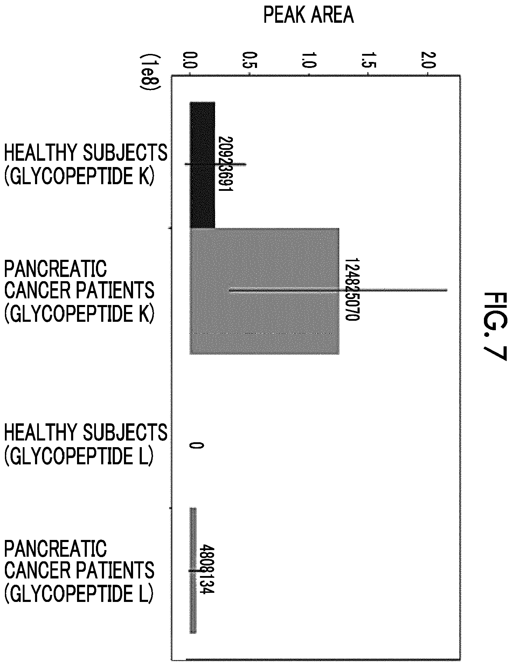

It was found that the expression levels of (i) glycopeptide K having the sugar chain represented by the formula 3-1' (not covered by the subject-matter of the claims, average peak area value in healthy subjects: 20923691, average peak area value in pancreatic cancer patients : 124825070) and (ii) glycopeptide L having the sugar chain represented by the formula 3-2' (not covered by the subject-matter of the claims, average peak area value in healthy subjects: 0, average peak area value in pancreatic cancer patients: 4808134), which are derived from thyroxine-binding globulin, are remarkably increased in pancreatic cancer patients as compared with healthy subjects, from Table 1 and Fig. 7. Particularly, it was found that glycopeptide L having the sugar chain represented by the formula 3-2' (not covered by the subject-matter of the claims) was not expressed at all in healthy subjects.

In addition, it was confirmed that the sugar chains represented by the formula 3-1' (not covered by the subject-matter of the claims) and the formula 3-2' (not covered by the subject-matter of the claims) are respectively bound to the asparagine residue at position 36 from the N-terminus of the amino acid sequence of thyroxine-binding globulin.

Further, the area under the curve values (AUC: curve area value) of the expression levels of glycopeptides A to L were calculated by receiver operating characteristic (ROC) curve analysis.

The results thereof are shown in Table 2 below. It should be noted that the AUC value is preferably 0.75 or more, and it is considered that when the AUC value is 0.80 or more, extremely high determination performance is obtained.

**[Table 2]**

| | AUC value | Glycopeptide | | | Binding site between peptide and sugar chain |
|---|---|---|---|---|---|
| | | peptide sequence | | sugar chain structure | |
| Glycopeptide A | 0,88 | vitronectin | NGSLFAFR (SEQ ID NO: 1) | Formula 1-1' | asparagine residue at position 169 from the N-terminus of the amino acid sequence of vitronectin |
| Glycopeptide B | 0,88 | | | Formula 1-2' | |
| Glycopeptide C | 0,88 | | | Formula 1-3' | |
| Glycopeptide D | 0,83 | | | Formula 2-1', Formula 2-1" | |
| Glycopeptide E | 0,83 | | | Formula 2-2' | |
| Glycopeptide F | 0,82 | | | Formula 3-1' | |
| Glycopeptide G | 0,82 | | | Formula 3-2' | |
| Glycopeptide H | 0,92 | complement C4-A | GLNVTLSSTGR (SEQ ID NO: 2) | Formula 4-1 | asparagine residue at position 1328 from the N-terminus of the amino acid sequence of complement C4-A |
| Glycopeptide I | 0,92 | inter-alpha trypsin inhibitor heavy chain H3 | NAHGEEKENL TAR (SEQ ID NO: 3) | Formula 5-1', Formula 5-1", Formula 5-2', Formula 5-2" | asparagine residue at position 580 from the N-terminus of the amino acid sequence of inter-alpha trypsin inhibitor heavy chain H3 |
| Glycopeptide J | 0,82 | leucine-rich alpha-2 glycoprotein | LPPGLLANFTLLR (SEQ ID NO: 4) | Formula 6-1 | asparagine residue at position 186 from the N-terminus of the amino acid sequence of leucine-rich alpha-2 glycoprotein |
| Glycopeptide K | 0,83 | thyroxine-binding globulin | VTACHSSQPNATL YK (SEQ ID NO: 5) | Formula 3-1' | asparagine residue at position 36 from the N-terminus of the amino acid sequence of thyroxine-binding globulin |
| Glycopeptide L | 0.80 | | | Formula 3-2' | |

As shown in Table 2, the glycopeptides A to L exhibited good AUC values.

It was found that whether a test sample (sample) has pancreatic cancer can be determined by using the glycopeptides A to L as markers from the above.

### Industrial Applicability

According to the pancreatic cancer determination marker, the pancreatic cancer determination method using the same, and the method for acquiring data for determining a pancreatic cancer of the present invention, it is possible to determine pancreatic cancer (diagnosis, testing) with a high accuracy (correctness and precision).

## Claims

1. A pancreatic cancer determination marker comprising a sugar chain represented by the formula 5 present in inter-alpha trypsin inhibitor heavy chain H3; 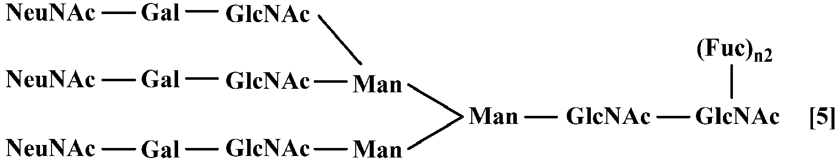 in the formula 5, n2 represents 0 or 1.

2. The marker according to claim 1, wherein the sugar chain is a sugar chain represented by the formula 5 bound to an asparagine residue at position 580 from a N-terminus of an amino acid sequence of inter-alpha trypsin inhibitor heavy chain H3.

3. A pancreatic cancer determination method, comprising:
measuring an amount of a sugar chain represented by the formula 5 present in inter-alpha trypsin inhibitor heavy chain H3 in a sample; 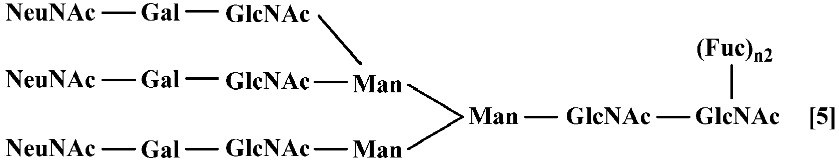
in the formula 5, n2 represents 0 or 1; and
determining pancreatic cancer based on an obtained measurement result.

4. The determination method according to claim 3, wherein the sugar chain is a sugar chain represented by the formula 5 bound to an asparagine residue at position 580 from a N-terminus of an amino acid sequence of inter-alpha trypsin inhibitor heavy chain H3.

5. The determination method according to claim 3, wherein the sample is whole blood, serum, or plasma.

6. A method for acquiring data for determining a pancreatic cancer, comprising measuring an amount of a sugar chain represented by the formula 5 present in inter-alpha trypsin inhibitor heavy chain H3; 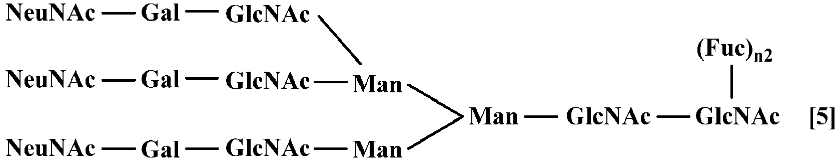 in the formula 5, n2 represents 0 or 1.

7. Use of a pancreatic cancer determination kit comprising a substance having an affinity for a sugar chain represented by the formula 5 present in inter-alpha trypsin inhibitor heavy chain H3; 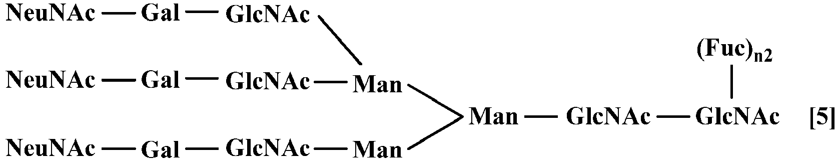 in the formula 5, n2 represents 0 or 1;
for the method selected from any one of claims 3 to 6.

## Patentansprüche

1. Marker zur Bestimmung von Bauchspeicheldrüsenkrebs, der eine durch die Formel 5 dargestellte Zuckerkette umfasst, die in der schweren Kette H3 des Inter-alpha-Trypsininhibitors vorhanden ist; 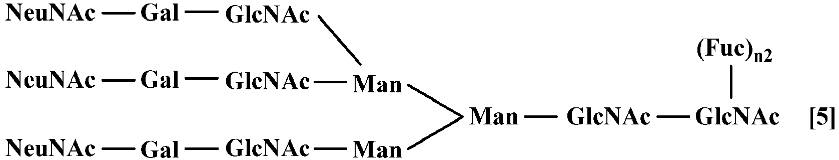 wobei in der Formel 5 n2 für 0 oder 1 steht.

2. Marker nach Anspruch 1, wobei die Zuckerkette eine durch die Formel 5 dargestellte Zuckerkette ist, die an einen Asparaginrest an Position 580 von einem N-Terminus einer Aminosäuresequenz der schweren Kette H3 des Inter-alpha-Trypsininhibitors gebunden ist.

3. Bauchspeicheldrüsenkrebsbestimmungsverfahren umfassend:
Messen in einer Probe einer Menge einer durch die Formel 5 dargestellten Zuckerkette, die in der schweren Kette H3 des Inter-alpha-Trypsininhibitors vorhanden ist; 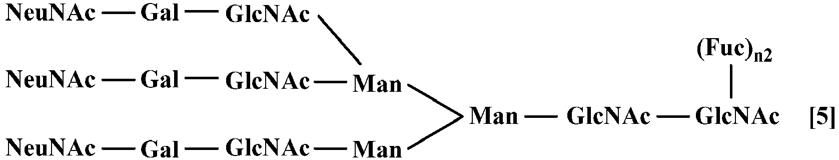
wobei in der Formel 5 n2 für 0 oder 1 steht; und
Bestimmen von Bauchspeicheldrüsenkrebs auf der Grundlage eines erhaltenen Messergebnisses.

4. Bestimmungsverfahren nach Anspruch 3, wobei die Zuckerkette eine durch die Formel 5 dargestellte Zuckerkette ist, die an einen Asparaginrest an Position 580 von einem N-Terminus einer Aminosäuresequenz der schweren Kette H3 des Inter-alpha-Trypsininhibitors gebunden ist.

5. Bestimmungsverfahren nach Anspruch 3, wobei die Probe Vollblut, Serum oder Plasma ist.

6. Verfahren zum Gewinnen von Daten zum Bestimmen eines Bauchspeicheldrüsenkrebses, umfassend das Messen einer Menge einer durch die Formel 5 dargestellten Zuckerkette, die in der schweren Kette H3 des Inter-alpha-Trypsininhibitors vorhanden ist; 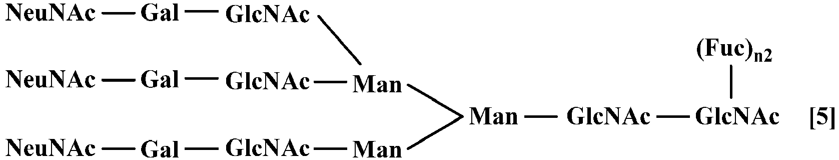 wobei in der Formel 5 n2 für 0 oder 1 steht.

7. Verwenden eines Bauchspeicheldrüsenkrebs-Bestimmungskits, das eine Substanz mit einer Affinität für eine durch die Formel 5 dargestellt Zuckerkette, die in der schweren Kette H3 des Inter-alpha-Trypsininhibitors vorhanden ist, umfasst; 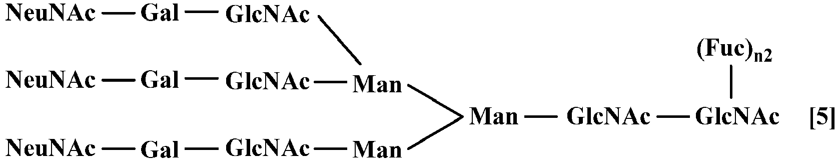
wobei in der Formel 5 n2 für 0 oder 1 steht;
für das aus einem der Ansprüche 3 bis 6 ausgewählte Verfahren.

## Revendications

1. Marqueur de détermination du cancer du pancréas comprenant une chaîne glucidique représentée par la formule 5 présente dans la chaîne lourde H3 de l'inhibiteur de la trypsine inter-alpha ; 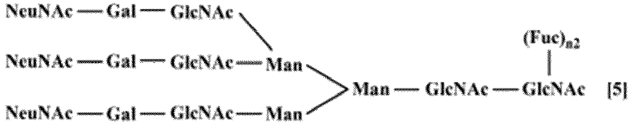 dans la formule 5, n2 représente 0 ou 1.

2. Marqueur selon la revendication 1, dans lequel la chaîne glucidique est une chaîne glucidique représentée par la formule 5 liée à un résidu d'asparagine en position 580 à partir d'une extrémité N-terminale d'une séquence d'acides aminés de la chaîne lourde H3 de l'inhibiteur de la trypsine inter-alpha.

3. Procédé de détermination du cancer du pancréas, comprenant :
la mesure d'une quantité d'une chaîne glucidique représentée par la formule 5 présente dans la chaîne lourde H3 de l'inhibiteur de la trypsine inter-alpha dans un échantillon ; 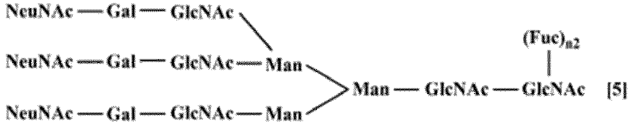 dans la formule 5, n2 représente 0 ou 1 ; et la détermination du cancer du pancréas sur la base d'un résultat de mesure obtenu.

4. Procédé de détermination selon la revendication 3, dans lequel la chaîne glucidique est une chaîne glucidique représentée par la formule 5 liée à un résidu d'asparagine en position 580 à partir d'une extrémité N-terminale d'une séquence d'acides aminés de la chaîne lourde H3 de l'inhibiteur de la trypsine inter-alpha.

5. Procédé de détermination selon la revendication 3, dans lequel l'échantillon est du sang total, du sérum ou du plasma.

6. Procédé d'acquisition de données pour déterminer un cancer du pancréas, comprenant la mesure d'une quantité d'une chaîne glucidique représentée par la formule 5 présente dans la chaîne lourde H3 de l'inhibiteur de la trypsine inter-alpha ; 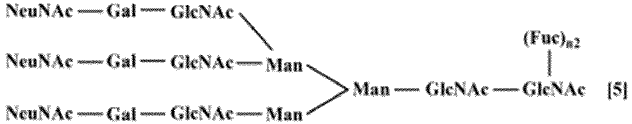 dans la formule 5, n2 représente 0 ou 1.

7. Utilisation d'un kit de détermination du cancer du pancréas comprenant une substance présentant une affinité pour une chaîne glucidique représentée par la formule 5 présente dans la chaîne lourde H3 de l'inhibiteur de la trypsine inter-alpha ; 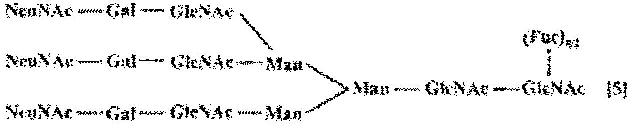
dans la formule 5, n2 représente 0 ou 1 ;
pour le procédé choisi parmi l'une quelconque des revendications 3 à 6.
